# EUROPEAN PATENT APPLICATION

(11) **EP 4 649 838 A1**
(43) Date of publication of application: **19.11.2025**
(21) Application number: 25730561.5
(22) Date of filing: 24.03.2025
(51) Int. Cl.: A23P 20/20, A23P 30/25, C12M 3/00, C12M 1/00, A23P 20/25

(54) **METHOD FOR PRODUCING ARTIFICIAL MEAT, AND APPARATUS FOR PERFORMING SAME**

(30) Priority: 25.03.2024 KR 20240040254
(71) Applicant: Tissenbiofarm Co., Ltd., Pohang-si, Gyeongsangbuk-do 37563 (KR)
(72) Inventor: HAN, Wonil, Pohang-si, Gyeongsangbut-do 37671 (KR)
(74) Representative: Derry, Paul Stefan
(86) International application number: PCT/KR2025/003714
(87) International publication number: WO 2025/206674

(57) **Abstract**

An apparatus for producing artificial meat using bio-ink is provided. The apparatus may comprise a plurality of modules and at least one processor configured to control the plurality of modules, wherein the plurality of modules include a monitoring module and an extrusion module, and the at least one processor is configured to control the monitoring module to maintain a predetermined condition of the bio-ink, and control the extrusion module to extrude and discharge at least a portion of the bio-ink.

## Description

### CROSS-REFERENCE TO RELATED APPLICATION

### BACKGROUND

### 1. Technical Field

The present disclosure relates to methods and apparatuses for producing artificial meat or artificial organs. More specifically, it pertains to a method and system for mass-producing artificial meat or organs in a short time by monitoring the state of bioink and extruding it in large quantities.

### 2. Discussion of the Related Art

The present disclosure relates to a method and an apparatus for producing artificial meat or an artificial organ using bioink. Artificial meat is an important future food source and an eco-friendly alternative that can contribute to suppressing global warming. The artificial meat addresses large-scale greenhouse gas emission problems occurring in conventional meat production processes and serves as a sustainable food production method. Artificial organs are also highly significant, contributing greatly to extending human health span, enhancing overall health, and strengthening bodily functions.

Existing methods for producing artificial meat and/or artificial organs (hereinafter collectively referred to as "artificial meat") have primarily relied on extruding bioink using 3D printing technology. However, this process has limitations in production speed and is inefficient for large-scale production. Moreover, artificial meat produced in this manner has limitations in reproducing the texture and cross-sectional shape of actual meat, which can work as a barrier for consumers to accept artificial meat as a substitute for the actual meat.

There is a need for technology development that can produce large amount of artificial meat within a short time by overcoming the limitations, accurately monitoring the state of bioink, and extruding bioink in large amounts.

### SUMMARY

An objective of the present disclosure is to provide a method of producing artificial meat that reproduces the texture and shape of actual meat within a short time. This is achieved by storing and monitoring bioink and extruding large amounts of bioink within a short time through an extrusion device.

The objective of the present disclosure is not limited to the above-described one. Other objectives may be clearly understood by those skilled in the art from the following descriptions and the accompanying drawings.

The embodiments of the present disclosure are not limited to ones described herein. Such embodiments are exemplary and various embodiment not described herein will be clearly understood by those skilled in the art.

According to an aspect of the present disclosure, an apparatus for producing artificial meat using bio-ink is provided. The apparatus may comprise a plurality of modules and at least one processor configured to control the plurality of modules, wherein the plurality of modules include a monitoring module and an extrusion module, and the at least one processor is configured to control the monitoring module to maintain a predetermined condition of the bio-ink, and control the extrusion module to extrude and discharge at least a portion of the bio-ink.

The present disclosure allows for the extrusion of large amounts of bioink at once through an artificial meat production apparatus, greatly improving the speed of the artificial meat producing process and enabling large-scale production. This provides a more realistic and economical approach to commercial production of artificial meat.

By producing artificial meat through the method of extruding large amounts of bioink in the form of fiber bundles, it is possible to produce the texture more closely to actual meat. This approach allows for artificial meat that is more like actual meat in terms of texture, shape, taste, and nutritional value, potentially increasing consumer acceptance.

The effects of the present disclosure are not limited to the effects described herein. The various effects can be clearly understood by those skilled in the art to which the present disclosure relates from the descritpons and the accompanying drawings.

### BRIEF DESCRIPTION OF THE DRAWINGS

FIG. 1 is a diagram illustrating an artificial meat producing system according to an embodiment of the present disclosure.
FIG. 2 is a diagram illustrating a configuration of a producing apparatus according to an embodiment of the present disclosure.
FIGS. 3 and 4 are diagrams illustrating a plurality of modules constituting a producing module according to an embodiment of the present disclosure.
FIGS. 5 and 6 are diagrams describing a pair of producing modules according to an embodiment of the present disclosure.
FIGS. 7 to 9 are diagrams illustrating a monitoring module according to an embodiment of the present disclosure.
FIGS. 10 and 11 are diagrams describing an extrusion module according to an embodiment of the present disclosure.
FIG. 12 is a cross-sectional view of a plunger of an extrusion module according to an embodiment of the present disclosure.
FIG. 13 is a diagram illustrating an extrusion operation of a plunger according to an embodiment of the present disclosure.
FIG. 14 is a diagram depicting a filling operation of a plunger according to an embodiment of the present disclosure.
FIG. 15 is a diagram showing a plunger of an extrusion module according to an embodiment of the present disclosure.
FIG. 16 is a diagram illustrating an extrusion operation and a filling operation of a plunger according to an embodiment of the present disclosure.
FIG. 17 is a diagram depicting a plunger of an extrusion module according to an embodiment of the present disclosure.
FIG. 18 is a diagram describing an extrusion operation and a filling operation of a plunger according to an embodiment of the present disclosure.
FIGS. 19 and 20 are diagrams illustrating a nozzle according to an embodiment of the present disclosure.
FIG. 21 is a diagram depicting an expandable producing module according to an embodiment of the present disclosure.
FIG. 22 is a diagram describing an arrangement and operation of an expandable producing module according to an embodiment of the present disclosure.
FIGS. 23 to 25 are diagrams illustrating a monitoring module included in an expandable producing module according to an embodiment of the present disclosure.
FIG. 26 is a diagram depicting a method of controlling an extrusion module based on user input according to an embodiment of the present disclosure.
FIG. 27 is a diagram illustrating an extrusion module according to another embodiment.
FIG. 28 is a diagram illustrating a transfer device according to an embodiment.
FIG. 29 is a diagram illustrating a flow of fluid in a transfer device according to an embodiment.
FIG. 30 and FIG. 31 are diagrams illustrating transfer efficiency when using a transfer device according to an embodiment.
FIG. 32 is a diagram illustrating a producing apparatus according to another embodiment.
FIG. 33 is a diagram illustrating a cell mixing device according to an embodiment.
FIG. 34 is a diagram illustrating the configuration of a cell mixing device according to an embodiment.
FIG. 35 is a diagram illustrating a detailed configuration of a stirring module according to an embodiment.
FIG. 36 and FIG. 37 are diagrams illustrating the operation of a cell mixing device according to an embodiment.
FIG. 38 and FIG. 39 are diagrams illustrating the operation method of multiple cell mixing modules and extrusion material composition modules according to an embodiment.
FIG. 40 is a diagram illustrating a post-processing module according to an embodiment.
FIG. 41 is a diagram illustrating the configuration of a crosslinking module according to an embodiment.
FIG. 42 is a diagram illustrating a method by which a processor controls the operation of the crosslinking module according to an embodiment.
FIG. 43 is a diagram illustrating the configuration of a culturing module according to an embodiment.
FIG. 44 is a diagram illustrating a method by which a processor controls the operation of the culturing module according to an embodiment.
FIG. 45 and FIG. 46 are diagrams illustrating methods by which a processor controls a post-processing procedure of an extrusion material according to an embodiment.
FIG. 47 is a diagram illustrating a method by which a processor controls and manages multiple modules constituting an automated system according to an embodiment.
FIG. 48 and FIG. 49 are diagrams illustrating methods by which a processor manages the state of a culturing module according to an embodiment.
FIG. 50 is a diagram illustrating a method by which a processor controls multiple containers included in a culturing module according to an embodiment.
FIG. 51 and FIG. 52 are diagrams illustrating an assembling module according to an embodiment.
FIG. 53 is a diagram illustrating the configuration of an assembling module according to an embodiment.
FIG. 54 through FIG. 56 are diagrams illustrating a first method of manufacturing artificial meat using an assembling module according to an embodiment.
FIG. 57 is a diagram illustrating a second method of manufacturing artificial meat using an assembling module according to an embodiment.
FIG. 58 is a diagram illustrating a method in which an assembling module operates in multiple working areas according to an embodiment.
FIGs. 59 through FIG. 61 are diagrams illustrating a third method of manufacturing artificial meat using an assembling module according to an embodiment.
FIGs. 62 to 64 are diagrams illustrating a method of manufacturing artificial meat with a predetermined pattern using an assembling module according to an embodiment.
FIG. 65 and FIG. 66 are diagrams illustrating a method of obtaining artificial meat by processing a unit structure including multiple layers using an assembling module according to an embodiment.
FIG. 67 is a diagram illustrating a cross-section of artificial meat produced by a producing apparatus according to an embodiment.

### DETAILED DESCRIPTION OF THE EMBODIMENTS

The present disclosure will be described more fully hereinafter with reference to the accompanying drawings, in which exemplary embodiments are shown. The disclosure may, however, be embodied in many different forms and should not be construed as limited to the embodiments set forth herein.

Throughout this specification, the same reference numbers refer to the same components. In the drawings of each embodiment, the components with the same function within the same scope and spirit of the embodiment will be described with the same reference numerals, and redundant descriptions thereof will be omitted.

Detailed descriptions of well-known functions or configurations related to the present disclosure will be omitted if their inclusion seems to make the subject matter of the present disclosure obscure. In addition, the numbers (e.g., first, second) used in the specification are merely symbols for differentiating one component from another. The numbers do not imply a sequential or hierarchical order unless the context clearly indicates otherwise.

The terms "module" and "unit" used herein are merely used for convenience of description and do not have any distinctive technical meaning, functions, or role. The terms "first" and "second" may be used to describe various components, but these terms are only for differentiation purposes.

As used herein, the singular forms "a," "an," and "the" are inclusive of their plural forms as well, unless the context clearly indicates otherwise. It will also understood that the symbol " / " (e.g., and/or) as used herein refers to and encompasses any and all possible combinations of one or more of the associated listed items. The terms "comprises," "comprising," "includes," and/or "including" specify the presence of stated features, steps, operations, elements, and/or components, but they do not preclude the presence or addition of one or more other features, steps, operations, elements, components, and/or groups thereof.

In the drawings, for convenience of explanation, the components may be exaggerated or reduced in size. For example, the size and thickness of each element shown in the drawings are illustrated for convenience of explanation. The present invention is not necessarily limited to the illustrations shown in the drawings. It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present.

The order of steps described in processes may be varied in embodiments. For instance, steps described sequentially may be performed simultaneously or in reverse order, where feasible.

For example, when a component is described as "electrically connected" in this specification, it includes both direct electrical connections and indirect electrical connections through intervening components.

This present disclosure describes methods for producing artificial meat and/or artificial organs. For the purposes of this disclosure, the terms "artificial meat" and "artificial organs" are interchangeable, as the production methods for both are substantially similar. Therefore, unless otherwise specified, the term "artificial meat" shall be understood to encompass both artificial meat and artificial organs throughout this specification. Where a distinction is necessary, it will be explicitly stated.

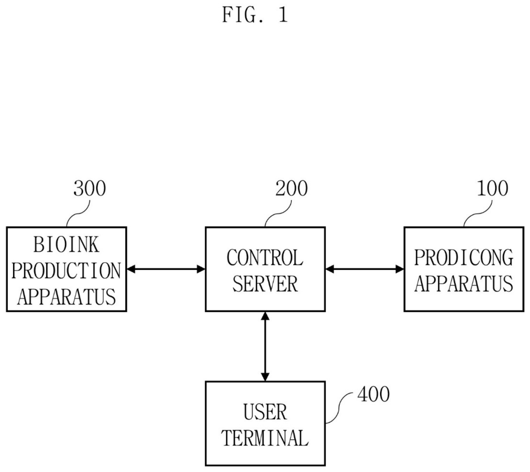

FIG 1 is a diagram illustrating an artificial meat producing system according to an embodiment of the present disclosure.

Referring to FIG. 1, the artificial meat producing system includes a producing apparatus 100, a control server 200, a bioink production apparatus 300, and a user terminal 400.

The bioink production apparatus 300 produces bioink used for artificial meat production. The producing apparatus 100 produces artificial meat using the bioink. The user terminal 400 receives user input for customized artificial meat production. The control server 200 controls at least one of the components in the system.

The bioink production apparatus 300 may produce one or more types of bioinks according to predetermined standards, including bioinks related to fat and protein. The producing apparatus 100 stores, monitors, and extrudes bioink produced by the bioink production apparatus 300. The producing apparatus 100 comprises multiple modules performing such functions (i.e., storing, monitoring, and/or extruding function), which will be described in detail below with reference to the drawings. Further, the producing apparatus 100 can either extrude or extract the bioink, depending on the specific process.

The control server 200 controls the functions of the bioink production apparatus 300 and/or the producing apparatus 100. The control server 200 communicates with the bioink production apparatus 300 and/or the producing apparatus 100, and it controls their operations based on signals received from the user terminal 400.

The user terminal 400 receives user input and information about desired artificial meat. The user can input the information (e.g., shape, texture, fat-to-protein ratio) via the user terminal 400, and the control server 200 uses the information to control the producing apparatus 100 for producing desired and customized artificial meat.

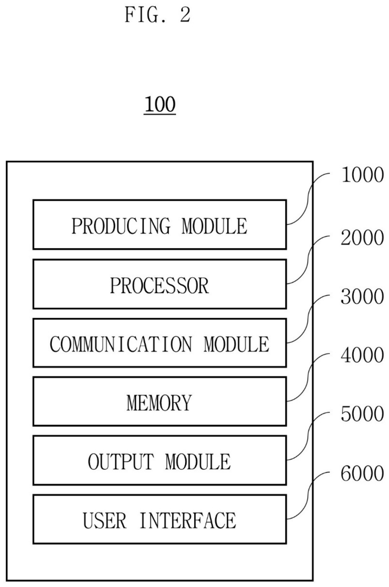

FIG. 2 illustrates the configuration of the producing apparatus 100 according to an embodiment of the present disclosure.

Referring to FIG. 2, the producing apparatus 100 may include at least one of modules: a producing module 1000, a processor 2000, a communication module 3000, a memory 4000, an output module 5000, and a user interface 6000.

The producing module 1000 performs an operation of storing, monitoring, extruding, and/or cross-linking bioink. It comprises multiple modules which will be described in detail later with reference to the drawings.

The processor 2000 controls the operation of the components in the producing apparatus 100. It executes software instructions to communicate with other components and interpret user instructions.

The communication module 3000 in the producing apparatus 100 enables information exchange with a server or an external device via a communication connection, and it facilitates functions such as bioink state monitoring, software updates, process optimization, and real-time remote control.

The memory 4000 stores commands for execution by the processor 2000 and information necessary for monitoring the bioink state.

The output module 5000 transmits information about producing events (e.g., process progress, warning messages, product quality evaluations) occurring during the production of artificial meat to the user. It may be implemented as visual or auditory output devices.

The user interface 6000 receives user input for controlling the apparatus operations. It may include various input tools such as a touchscreen, a button, keyboard, and a mouse, allowing users to start/stop producing operations and set parameters like bioink type, extrusion speed, and temperature.

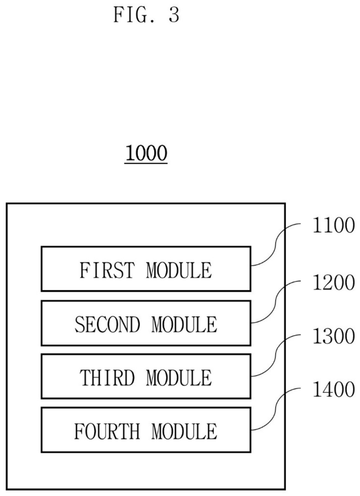

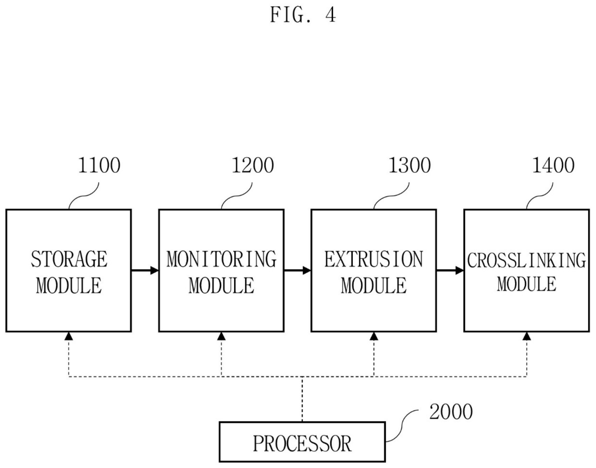

FIGS. 3 and 4 illustrate the components of a producing module according to an embodiment of the present disclosure.

Referring to FIG. 3, the producing module 1000 comprises a plurality of functional modules, including a first module 1100, a second module 1200, a third module 1300, and a fourth module 1400. Each module performs an operation of storing, monitoring, extruding, and crosslinking bioink.

Referring to FIG. 4, the first module 1100 functions as a storage module, the second module 1200 as a monitoring module, the third module 1300 as an extrusion module, and the fourth module 1400 as post-processing module.

The storage module 1100 is configured to store bioink and may be implemented as a bulk tank with sufficient internal capacity to accommodate large volumes of bioink.

The monitoring module 1200 is designed to monitor the state of the bioink based on at least one parameter. The monitoring module 1200 may monitor the temperature or pH value of the bioink. Further details of the monitoring module 1200 will be described below with reference to the drawings.

The extrusion module 1300 is responsible for extruding bioink. It utilizes a syringe-based mechanism to apply pressure and extrude the bioink in the form of fiber bundles. A more detailed explanation of this process will be described in later sections.

The post-processing module 1400 comprises an internal space capable of accommodating a predetermined volume of crosslinking liquid. This module may be implemented as a tank or water bath with an internal space in which the crosslinking liquid may be stored. The crosslinking liquid may be an aqueous solution including chemicals, such as calcium chloride (CaCl2), or liquid with a predetermined temperature like tap water or purified water.

The post-processing module 1400 incorporates at least one sensor, such as an electrical conductivity sensor, to monitor the state of the crosslinking liquid. The post-processing module 1400 may measure the contamination density of the inner space based on the electrical conductivity sensor. Based on sensor readings, the processor 2000 may initiate circulation and replacement of the crosslinking liquid if contamination levels exceed a specified threshold.

For maintenance purposes, the post-processing module 1400 is designed to be detachable from the producing module 1000. It may be implemented as a separable cart because the crosslinking liquid stored in the post-processing module 1400 needs a periodic replacement or needs to be replaced if it satisfies a predetermined standard. The module may feature a transparent case for visual inspection.

The post-processing module 1400 includes a pump and pipeline network to operate a circulation system. The post-processing module 1400 enables replacement of the crosslinking liquid, using the circulation system, when a predetermined time or standard is met.

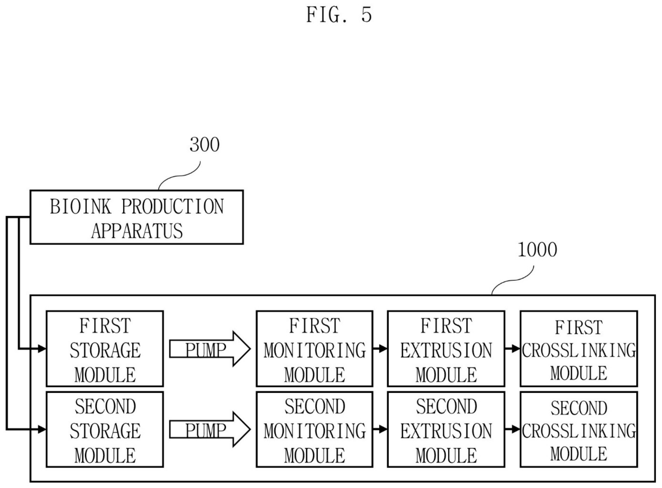

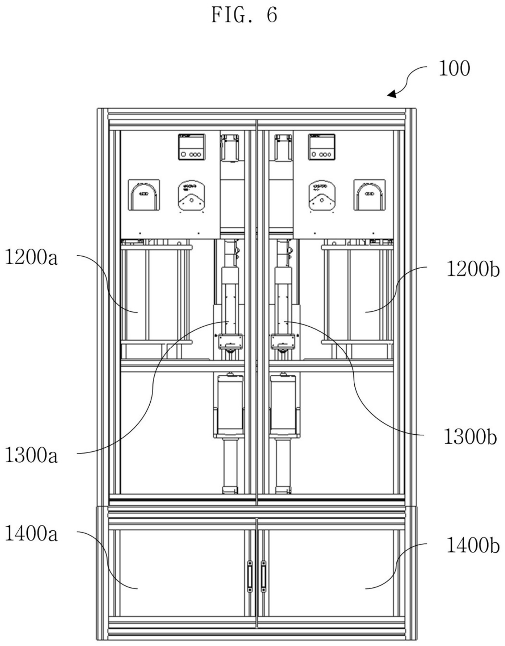

FIGS. 5 and 6 depict a pair of producing modules according to an embodiment of the present disclosure.

Referring to FIGS 5 and 6, the producing module 1000 may include a plurality of storage, monitoring, extrusion, and post-processing module to process different types of bioink.

In one embodiment, the type of bioink may be plural. For example, the bioink may include a first bioink related to fat and a second bioink related to protein.

The storage module 1100 includes a first storage module for the fat-related bioink and a second storage module for the protein-related bioink. The monitoring module 1200, extrusion module 1300, and post-processing module 1400 each comprise paired components to handle both types of bioink. For instance, the monitoring module 1200 may include a first monitoring module 1200a for monitoring the first bioink and a second monitoring module 1200b for monitoring the second bioink. The extrusion module 1300 may include a first extrusion module 1300a for extruding the first bioink and a second extrusion module 1300b for extruding the second bioink. The post-processing module 1400 may include a first post-processing module 1400a for crosslinking the first bioink and a second post-processing module 1400b for crosslinking the second bioink.

The processing sequence for each bioink type is as follows: bioink produced by the bioink production apparatus 300 is stored in its respective storage module (i.e., the first bioink in the first storage module, the second bioink in the second storage module). The corresponding monitoring module (1200a or 1200b) monitors the state of each bioink as it is pumped through the system. The respective extrusion module (1300a or 1300b) then extrudes the bioink in fiber bundle form. Finally, the appropriate post-processing module (1400a or 1400b) crosslinks the extruded bioink.

The above-descried pair of storage modules 1100, monitoring modules 1200, extrusion modules 1300, and post-processing module 1400 may be arranged symmetrically and can be controlled in parallel or independently, enabling simultaneous processing of multiple bioink types.

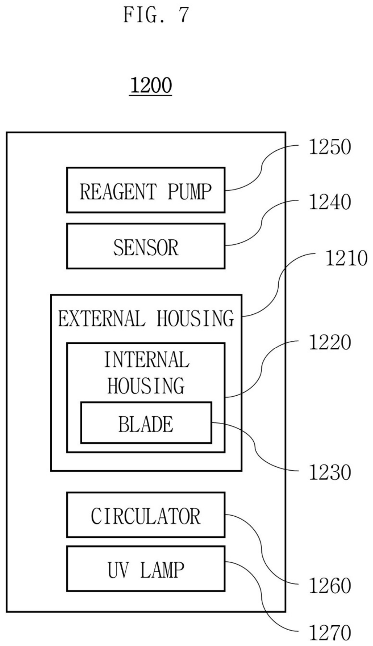

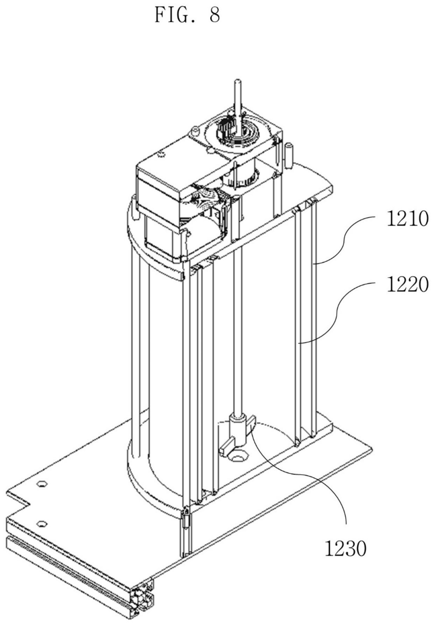

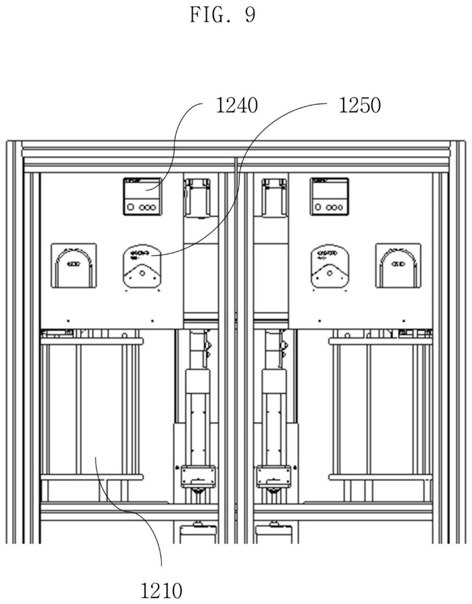

FIGS. 7-9 illustrate a monitoring module 1200 according to an embodiment of the present disclosure.

Referring to FIGS 7 to 9, the monitoring module 1200 comprises an external housing 1210 and an internal housing 1220.

The monitoring module 1200 may include an internal space for accommodating bioink. The internal space may be formed based on at least one of the external housing 1210 and internal housing 1220. The internal housing 1220 may be positioned within the external housing 1210. The monitoring module 1200 may accommodate bioink in the internal space.

The monitoring module 1200 further include a blade 1230. The blade 1230 may be located within the internal space of the internal housing 1220. The blade 1230 has an impeller shape and is configured to agitate the bioink by rotating under power.

The blade 1230 may be spaced apart from the inner surface of the internal housing 1220 by a predetermined distance. When rotated, the blade 1230 forms a first radius that is smaller than the second radius of the internal housing 1220.

When the first radius is formed within the predetermined numerical range, more efficient agitation may proceed. For instance, the difference between the first radius and the second radius may be about 10 mm. In this case, the blade 1230 is more closely attached to the inner surface of the internal housing 1220 to rotate, thereby enabling more efficient agitation of the bioink.

The shape of the blade 1230 may be provided in various forms (e.g., U-shape) other than the shape illustrated in the drawing. When the blade 1230 is provided in a U-shape, the area attached to the inner surface of the internal housing 1220 among the blade 1230 components increases, thereby more effectively performing the agitation of the bioink.

The monitoring module 1200 further include at least one sensor 1240 for monitoring the state of the bioink stored in the internal housing 1220. The sensor 1240 may include a temperature sensor measuring the temperature of the bioink and/or a pH sensor (or pH measurement device) measuring the pH value.

The monitoring module 1200 includes a reagent pump 1250 for controlling the state of the bioink stored in the internal housing 1220. The monitoring module 1200 may monitor the pH value of the bioink based on the at least one sensor 1240 and adjust the pH of the bioink using the reagent pump 1250 when the measured pH value deviates from a predetermined condition.

The potential of hydrogen (pH) of the bioink may be a critical factor in determining the quality of artificial meat. As it is important to monitor and control the pH value of the bioink to maintain an appropriate level (e.g., neutral), the producing apparatus 100 can monitor and control the pH value of the stored bioink through the monitoring module 1200 to ensure it is maintained at an appropriate level.

The monitoring module 1200 includes a dual jacket structure formed by the outer housing 1210 and the inner housing 1220. The monitoring module 1200 may have a multi-layer space, corresponding to the dual jacket structure, which can be formed between the outer housing 1210 and the inner housing 1220.

The monitoring module 1200 includes a circulator 1260 for maintaining the state of the bioink in accordance with predetermined conditions. The circulator 1260 includes a fluid circulation mechanism to control and maintain the temperature of the bioink constant.

The monitoring module 1200 may control the state of the bioink stored in the housing through the circulator 1260. For example, the monitoring module 1200 may control the temperature of the bioink stored in the inner housing 1220 by circulating the fluid to the multi-layer space using the circulator 1260.

More specifically, the monitoring module 1200 may monitor the temperature of the bioink measured by the at least one sensor 1240, and it may control the temperature of the bioink through the circulator 1260 when it is determined that the measured temperature value deviates from the predetermined condition.

The monitoring module 1200 includes a heating block for controlling the temperature of the bioink stored in the housing. For example, the monitoring module 1200 may monitor the temperature of the bioink measured by the at least one sensor 1240, and it may control the heating block to maintain the measured temperature constantly. The heating block may refer to a device that transfers heat generated from the heating element to the block to compensate for the temperature.

The monitoring module 1200 includes a Peltier system, including a Peltier element. The Peltier system, which may be electrical or water-cooled, can control the temperature of the bioink. The monitoring module 1200 may control the state of the bioink stored in the housing using the Peltier system. For example, the monitoring module 1200 may monitor the temperature of the bioink measured by the at least one sensor 1240 and control the Peltier system to maintain the measured temperature constant. The monitoring module 1200 may adjust the parameters as needed to maintain predetermined conditions critical for artificial meat quality.

The monitoring module 1200 may control the state of the bioink through a combination of at least one of the circulating system, the heating block, and the Peltier system based on the dual jacket structure described above.

The monitoring module 1200 includes a UV lamp 1270 disposed in either the outer housing 1210 or the inner housing 1220 for sterilizing the bioink stored in the internal space.

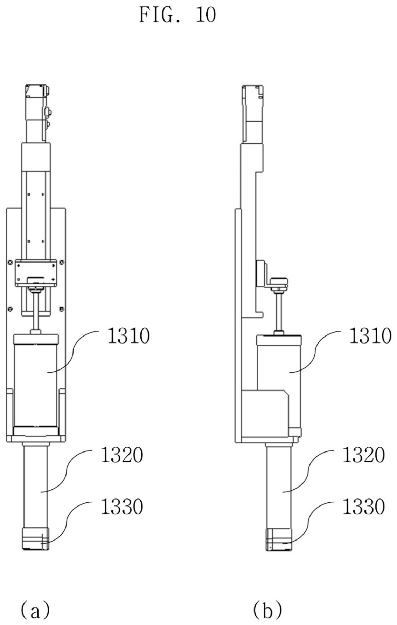

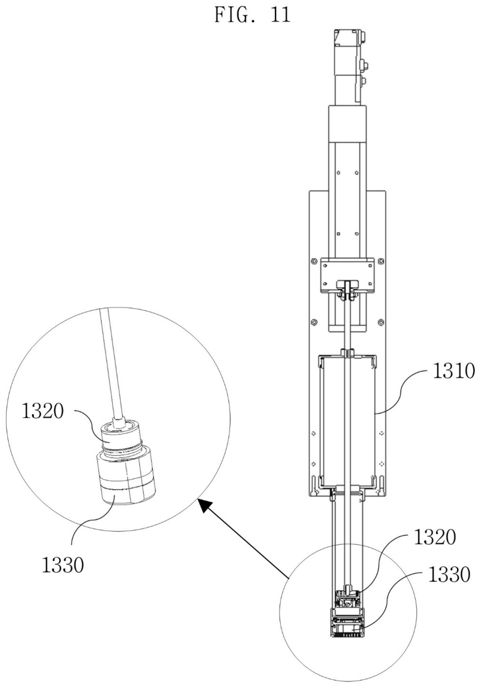

FIGS. 10 and 11 illustrate an extrusion module according to an embodiment of the present disclosure. The extrusion module 1300 comprises a device for extruding multiple fiber bundles by applying pressure to bioink.

The extrusion module 1300 includes a syringe 1310 forming an inner space, a plunger 1320 disposed within the inner space of the syringe, and a nozzle 1330 disposed at an end of the plunger.

The inner space of syringe 1310 receives bioink. At least a portion of the bioink stored in the inner housing 1220 of monitoring module 1200 may be transferred to the inner space of syringe 1310 by an external force.

The bioink accommodated in syringe 1310 may be extruded and extracted as fiber bundles. Plunger 1320 is configured to move vertically (i.e., rising or falling) within syringe 1310, powered by an actuator. The vertical movement of plunger 1320 causes extrusion and extraction of at least a portion of the bioink.

A nozzle 1330, disposed at the end of plunger 1320, comprises multiple holes or slits of predetermined diameters. By controlling the vertical movement of plunger 1320, the extrusion module 1300 extrudes and extracts at least a portion of the bioink as fiber bundles through the holes of nozzle 1330.

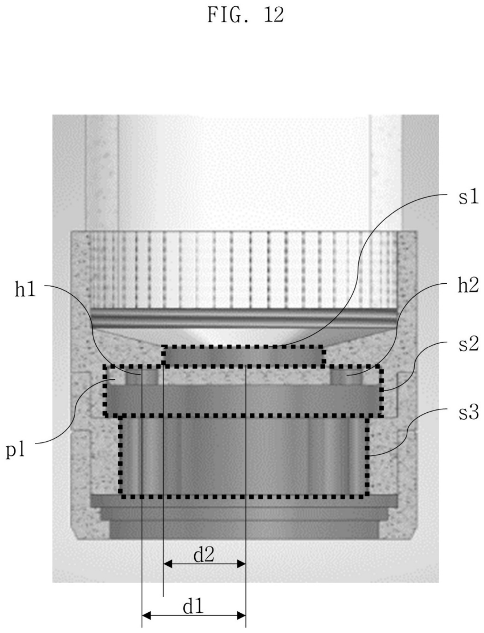

FIG. 12 depicts a cross-sectional view of plunger 1320 of an extrusion module according to an embodiment of the present disclosure. Referring to FIG 12, the plunger 1320 may comprise multiple internal spaces.

The plunger 1320 includes a first internal space S1, a second internal space S2; and a third internal space S3. Here, the diameter (or width) of first internal space S1 is smaller than that of second internal space S2. For descriptive purposes, S1, S2, and S3 may be referred to as first, second, and third layers, respectively. For ease of description, the first to third layers will be mixed with each other as the first to third internal spaces.

A plate Pl, comprising at least one hole, is disposed within second internal space S2. Plate Pl moves vertically (i.e., movig up and down) within S2 in response to the vertical movement of plunger 1320. The configuration of the hole(s) in plate Pl may vary.

Plate P1 may include a first hole H1 and a second hole H2, positioned at a first distance D1 from the center of plate Pl. The radius of first internal space S1 is defined as a second distance D2, where D1 is greater than D2. H1 and H2 are located outside a first radius range centered on plate Pl, where the first radius range is equal to or greater than the radius of the first internal space S1.

The fluid passage within plunger 1320 may be opened or closed by the vertical movement of plate Pl. The following describes the mechanism of blocking or opening the fluid passage based on the movement of plunger 1320 and plate Pl.

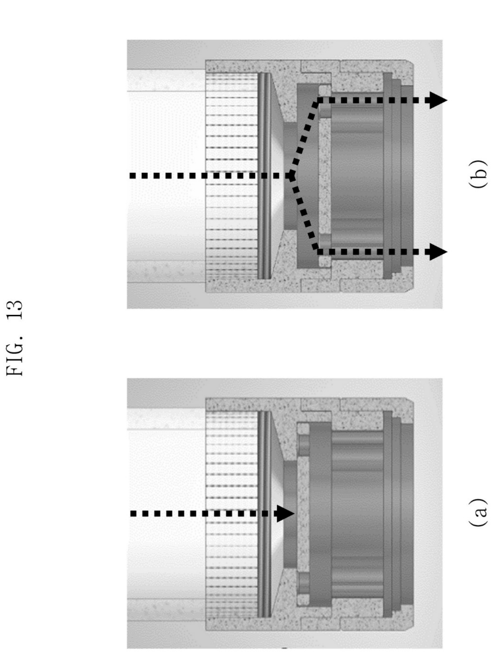

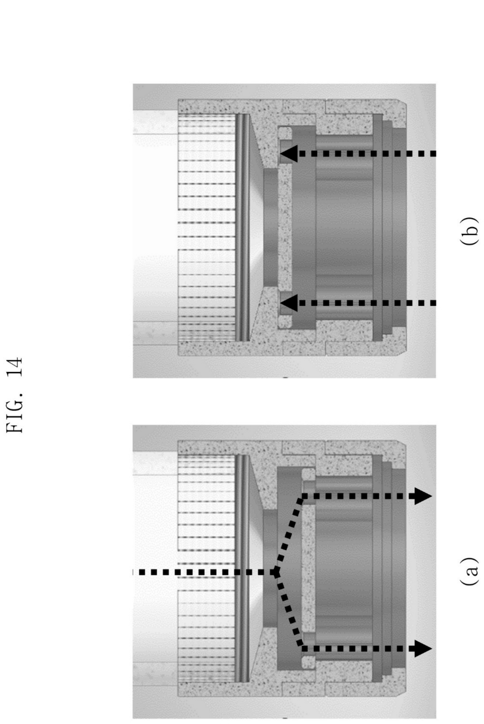

FIGS. 13 and 14 illustrate the extrusion and filling operations of the plunger, respectively according to an embodiment of the present disclosure. Referring to FIG 13 and 14, a movement may occur in the plate located within the second internal space corresponding to the movement of the plunger 1320, the movement direction of the bioink may be determined accordingly.

Referring to FIG 13, when the extrusion operation of the plunger 1320 is performed, the plate may move in a first direction within syringe 1310. This movement causes plate to move in the first direction, allowing bioink stored in the syringe to pass through the hole(s) in plate and move in the first direction. The extrusion operation may be an operation in which the plunger 1320 moves in the first direction inside the syringe 1310.

When plunger 1320 performs the extrusion operation, the plate moves in the first direction, so that the first, second, and third inner spaces (S1, S2, and S3) of the plunger 1320 may be fluidly connected.

When the extrusion operation of the plunger 1320 is performed, as shown in Fig. 13(b), the plate moves in the first direction due to pressure, allowing at least a portion of the bioink stored inside the syringe 1310 to pass through the hole formed in the plate and move in the first direction. The extrusion operation may be an operation in which the plunger 1320 moves in the first direction within the syringe 1310.

When the extrusion operation of the plunger 1320 is performed, as shown in Fig. 13(b), the plate moves in the first direction due to pressure, which may open a first movement passage allowing at least a portion of the bioink stored inside the syringe 1310 to move to the post-processing module 1400. Here, the first direction corresponds to the direction of arrows in FIGS. 13(a) and 13(b).

Referring to FIG. 14, during the filling operation of plunger 1320, the plate moves in the second direction, blocking bioink movement in the second direction. The filling operation comprises plunger 1320 moving in the second direction within syringe 1310. Here, the second direction may be the direction of the arrow in FIG. 14(b).

When the filling operation of the plunger 1320 is executed, the plate's movement in the second direction may fluidically block the first inner space S1 from the second inner space S2 of plunger 1320.

As shown in FIG. 14(b), pressure-induced plate movement in the second direction causes the plate's closed area to shield the first internal space S1 of plunger 1320. This shielding action prevents bioink backflow into syringe 1310. The closed area refers to the non-perforated area of the plate's surface.

The filling operation of the plunger 1320 causes the plate to move in the second direction under pressure, as shown in FIG. 14(b), thereby blocking the aforementioned first movement passage.

During the filling operation of the plunger (1320), the shielding action of the plunger (1320) may be performed, thereby blocking the first passage. This blockage allows the internal space of the syringe (1310) to maintain a vacuum state. Simultaneously, the crosslinking of the bioink contained within the syringe (1310) can be avoided by preventing the influx of crosslinking liquid stored in the post-processing module (1400) into the syringe (1310).

By repeatedly performing the extrusion and the filling operations of plunger 1320, the system can repeatedly extrude a portion of the bioink from syringe 1310 by extruding it externally (e.g., to post-processing module 1400) and subsequently refill the syringe with bioink.

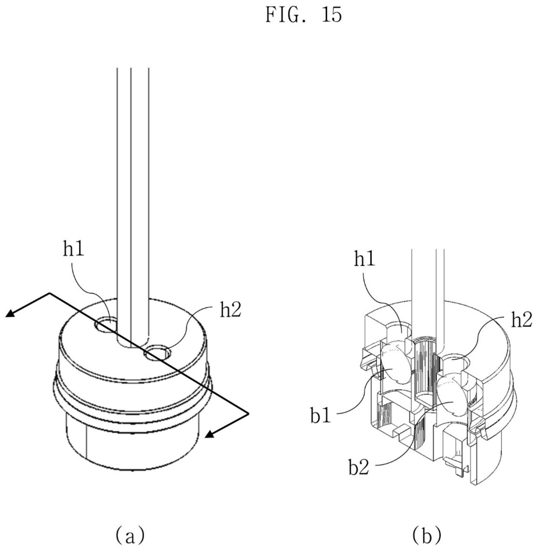

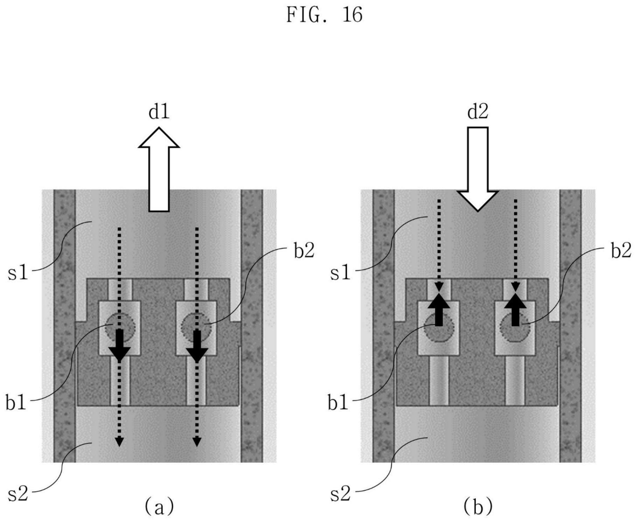

FIG. 15 depicts an alternative plunger embodiment for the extrusion module. FIG. 16 illustrates the extrusion and filling operations of a plunger according to an embodiment of the present disclosure.

Referring to FIG. 15(a), plunger 1320 of extrusion module 1300 may employ a ball-spring mechanism. The plunger 1320 comprises at least one fluid passage hole, through which bioink may flow. For instance, plunger 1320 may include a first hole h1 and a second hole h2 for bioink movement.

As showin in FIG. 15(b), the plunger 1320 of extrusion module 1300 includes at least one ball and spring. The ball and spring reside inside the plunger housing, positioned along the fluid flow path through the holes.

The ball and spring are typically arranged in pairs. For example, the plunger 1320 may include a first ball b1 on the fluid path formed by first hole h1, a second ball b2 on the fluid path formed by second hole h2, with corresponding first and second springs positioned relative to balls b1 and b2, respectively.

Referring to FIG. 16, the plunger's vertical motion induces movement in at least one ball (b1 and b2) within plunger 1320, thereby opening or closing the fluid movement passage.

As illustrated in FIG. 16(a), when plunger 1320 moves in the first direction d1 (upward; lifting operation), pressure displaces at least one ball (b1 and b2) inside plunger 1320 in the second direction d2, with d1 and d2 being opposite directions.

This ball displacement in direction d2 fluidically connects the first internal space s1 and second internal space s2 of syringe 1310, which are separated by plunger 1320. This connection opens the movement passage within plunger 1320, allowing bioink stored in syringe 1310 to flow in the second direction through the newly opened movement passage.

Thus, plunger 1320's movement in the first direction facilitates the filling operation by enabling bioink transfer from syringe 1310 in the second direction.

As depicted in FIG. 16(b), when plunger 1320 moves in the second direction d2 (downward; falling operation), pressure forces at least one ball (b1 and b2) inside plunger 1320 to move in the first direction d1, with d1 and d2 being opposite directions.

This ball movement in direction d1 fluidically blocks the first inner space s1 from the second inner space s2 within syringe 1310. Consequently, the movement passage inside plunger 1320 is blocked, preventing bioink in syringe 1310 from flowing in the second direction through the passage.

Therefore, when plunger 1320 moves in the second direction, the bioink stored in the syringe 1310 cannot flow in the second direction, enabling bioink extrusion through nozzle 1330. Specifically, this plunger movement allows for extrusion of bioink from the second internal space s2 while simultaneously preventing bioink introduction from the first internal space s1 into the second internal space s2.

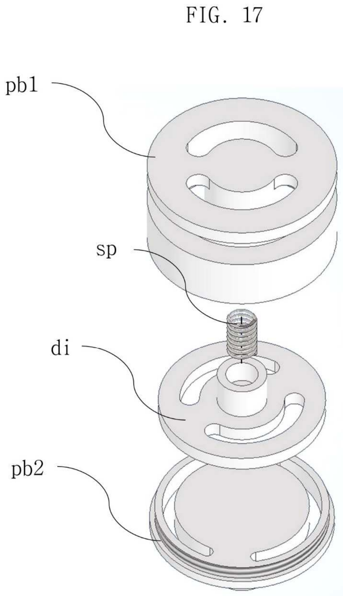

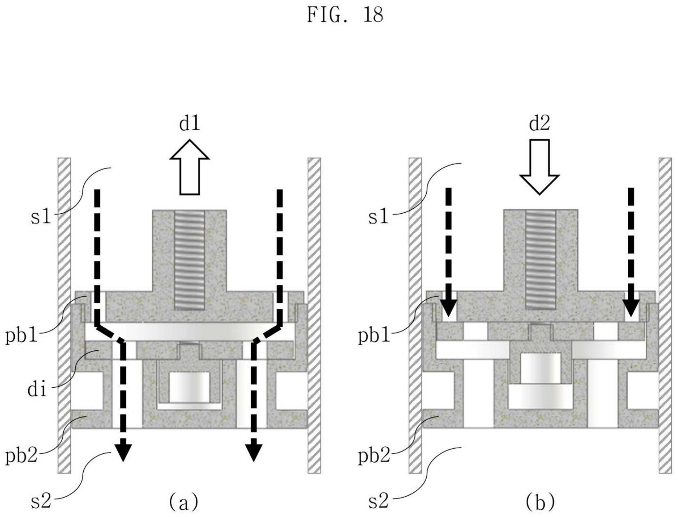

FIG. 17 illustrates a plunger of an extrusion module according to an embodiment of the present disclosure. FIG. 18 depicts the extrusion and filling operations of the plunger according to an embodiment of the present disclosure.

Referring to FIG 17, the plunger 1320 comprises a first plunger body pb1 and a second plunger body pb2, each having at least one aperture (i.e., hole). The first and second plunger bodies are configured to be detachably coupled.

A disc di is disposed within an internal space formed between the first plunger body pb1 and the second plunger body pb2. The disc di includes at least one aperture aligned with a corresponding aperture in the second plunger body pb2.

The pluner 1320 may include a spring sp, enabling the disc di to move within the internal space in response to plunger movement.

As illustrated in FIG. 18, the disc di moves in response to the plunger's vertical displacement, thereby opening or closing a fluid passage within the plunger 1320.

Referring to FIG. 18(a), during the plunger's upward movement (first direction d1), the disc di is displaced downward (second direction d2) due to pressure, opposite to the plunger's movement direction.

As the disk di moves in the second direction d2, the first internal space s1 and the second internal space s2 whitin the syringe 1310, which are separated by the plunger 1320, may become fluidly connected. This fluid connection opens the movement passage within the plunger (1320). Consequently, at least a portion of the bioink stored in the syringe 1310 can move through the movement passage in the second direction.

When the plunger moves internal spaces in the syringe 1310 in the first direction, the bioink stored in the syringe 1310 can move in the second direction, and thus the operation of filling the bioink can be performed.

As depicted in FIG. 18(b), during the plunger's downward movement (second direction d2), the disc di is displaced upward (first direction d1), opposite to the second direction d2.

This movement blocks fluid transfer between the first and second internal spaces which are separated by the plunger 1320, closing the fluid passage and preventing bioink backflow from the second to the first internal space. As the first internal space s1 and the second internal space s2 are blocked, the movement passage inside the plunger 1320 may be blocked and the bioink stored inside the syringe 1310 cannot move in the second direction d2.

When the plunger 1320 moves in the second direction d2 (downward movement), the bioink stored within the syringe 1310 is prevented from moving in the second direction d2, thereby facilitating the bioink extrusion through the nozzle 1330. Specifically, the plunger's downward movement extrudes at least a portion of the bioink stored in the second internal space s2. Simultaneously, this downward movement blocks the flow of bioink from the first internal space s1 into the second internal space s2.

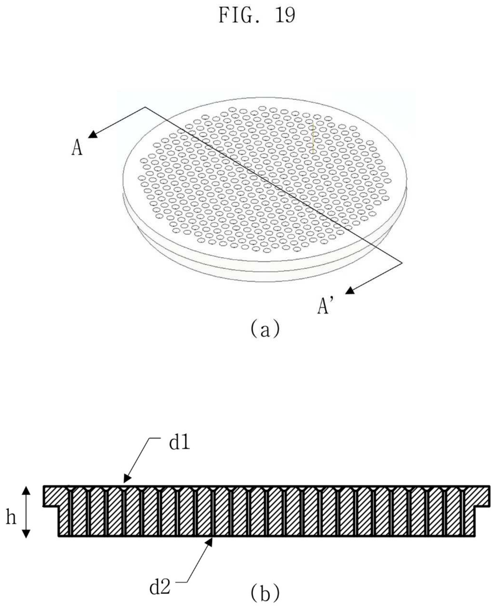

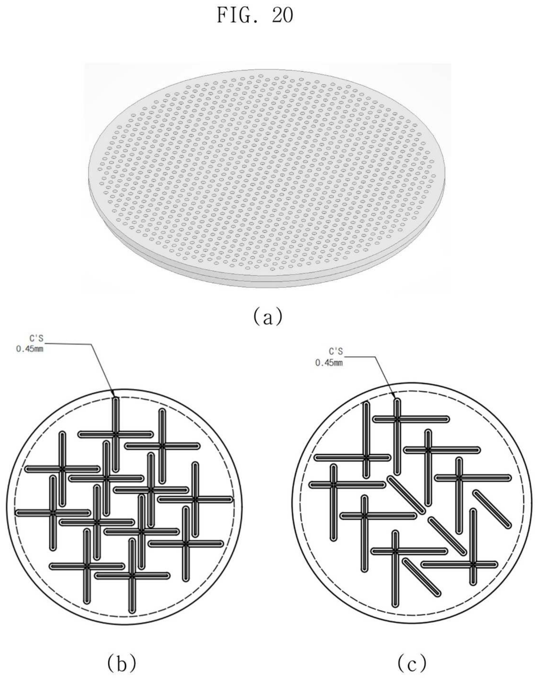

FIGS. 19 and 20 illustrate nozzle configurations according to an embodiment of the present disclosure.

Referring to FIG. 19(a), the nozzle 1330 comprises a plurality of holes arranged in a predetermined pattern. The holes in the nozzle 1330 may be spaced apart at uniform intervals or by the same distance.

As shown in FIG. 19(b), the nozzle 1330 includes multiple longitudinally-oriented holes of a predetermined height. These holes serve as fluid passages.

The multiple holes in nozzle 1330 have a Y-shaped cross-section. Each hole has an inlet with a first radius d1 and an outlet with a second radius d2. In this case, d1 may be greater than d2. Illustratively, the inlet diameter in one surface is 0.45 cm and the outlet diameter in the other surface is 0.3 cm.

Referring to FIG. 20, while the nozzle holes may be circular, they are not limited to this shape and may alternatively be polygonal, linear, or slit-shaped. The shape of the extruded bioink varies based on the hole configuration (i.e., shape of the hole).

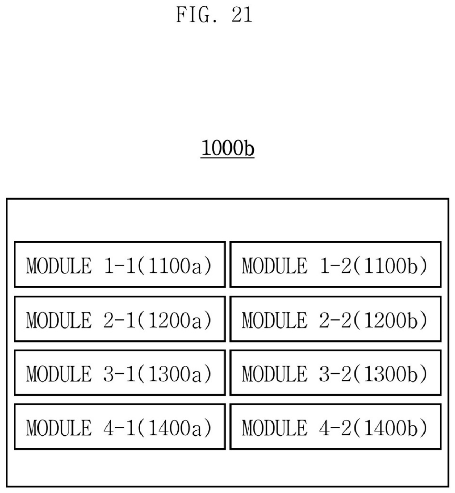

FIG. 21 depicts an expandable producing module according to an embodiment of the present disclosure. Referring to FIG. 21, the expandable producing module 1000b may include a plurality of modules.

The producing module 1000b includes storage modules 1100a and 1100b, monitoring modules 1200a and 1200b, extrusion modules 1300a and 1300b, and post-processing modules 1400a and 1400b (i.e., a 1-1 module 1100a, a 1-2 module 1100b, a 2-1 module 1200a, a 2-2 module 1200b, a 3-1 module 1300a, a 3-2 module 1300b, a 4-1 module 1400a, and a 4-2 module 1400b). These paired modules correspond functionally to the single modules described in reference to FIG. 4.

The 1-1 module 1100a and the 1-2 module 1100b may correspond to the storage module 1100 described in FIG 4. For example, the 1-1 module 1100a may be a first storage module, and the 1-2 module 1100b may be a second storage module. The first storage module and the second storage module may correspond to the storage module 1100 as shown in FIG 4 with a shape or functional configuration, and thus redundant descriptions will be omitted.

The 2-1 module 1200a and the 2-2 module 1200b may correspond to the monitoring module 1200 described in FIG 4. For example, the 2-1 module 1200a may be a second monitoring module, and the 2-2 module 1200b may be a second monitoring module. The first monitoring module and the second monitoring module may correspond to the monitoring module 1200 as showin in FIG 4 with a shape or functional configuration, and thus redundant descriptions will be omitted.

The 3-1 module 1300a and the 3-2 module 1300b may correspond to the extrusion module 1300 described in FIG 4. For example, the 3-1 module 1300a may be a first extrusion module, and the 3-2 module 1300b may be a second extrusion module. The first extrusion module and the second extrusion module may correspond to the extrusion module 1300 a shown in FIG 4 with a shape or functional configuration, and thus redundant descriptions will be omitted.

The 4-1 module 1400a and the 4-2 module 1400b may correspond to the post-processing module 1400 described in FIG 4. For example, the 4-1 module 1400a may be a first post-processing module, and the 4-2 module 1400b may be a second post-processing module. The first post-processing module and the second post-processing module may correspond to the post-processing module 1400as shown in FIG 4 with a shape or functional configuration, and thus redundant descriptions will be omitted.

The producing module 1000b may comprise a first unit and a second unit. The first unit includes the 1-1 module 1100a, the 2-1 module 1200a, the 3-1 module 1300a, and the 4-1 module 1400a. The second unit includes the 1-2 module 1100b, the 2-2 module 1200b, the 3-2 module 1300b, and the 4-2 module 1400b. The first unit and the second unit may process a different bioink type. For example, the first unit may handle fat-related bioink while the second unit processes protein-related bioink.

The producing module 1000b includes a plurality of modules configured in a pair of combinations. This paired configuration enables simultaneous mass production of artificial meat and/or organs.

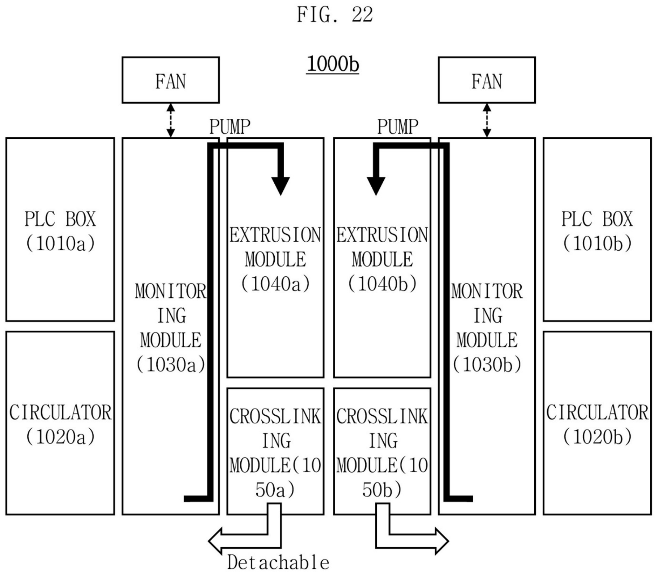

FIG. 22 illustrates the arrangement and operation of the expandable producing module 1000b according to an embodiment of the present disclosure.

Referring to FIG 22, the expandable producing module 1000b includes a pair of Programmable Logic Controller (PLC) boxes. The expandable producing module 1000b includes a first PLC box 1010a and a second PLC box 1010b.

The PLC box may be used to automate and control various devices and modules in the producing process. The producing module 1000b may control at least one operation of the circulator, the monitoring module, the extrusion module, and the post-processing module through a pair of PLC boxes. Users can pre-program operations of circulators, monitoring modules, extrusion modules, and post-processing module through these PLC boxes.

The producing module 1000b further comprises a pair of circulators, a first circulator 1020a and a second circulator 1020b. The pair of circulators may correspond to circulator 1260 descried in FIG. 7. For example, the first circulator 1020a may control the temperature of the bioink stored in the inner space of the first monitoring module 1030a by circulating the fluid to the multi-layer space in the first monitoring module 1030a. Similarly, the second circulator 1020b may control the temperature of the bioink stored in the inner space of the second monitoring module 1030b by circulating the fluid to the multi-layer space in the second monitoring module 1030b.

The producing module 1000b may include a pair of monitoring modules, a first monitoring module 1030a and a second monitoring module 1030b. The pair of monitoring modules may correspond to the monitoring module 1200 described in FIG 7.

The producing module 1 000b may include a pair of extrusion modules, a first extrusion module 1040a and a second extrusion module 1040b. The pair of extrusion modules may correspond to the extrusion module 1300 described in FIG 7. Bioink is transferred from monitoring modules to extrusion modules via pump. Then, the extrusion modules extrude the bioink stored in the inner space in the form of a fiber bundle.

The producing module 1000b may include a pair of post-processing modules, a first post-processing module 1050a and a second post-processing module 1050b. The pair of post-processing modules may correspond to the post-processing module 1400 described in FIG 7. The bioink extruded by the pair of extrusion modules may enter the pair of post-processing modules. The crosslinking reaction may occur by a crosslinking solution in the pair of post-processing modules. These post-processing modules are detachably coupled from the producing module 1000b.

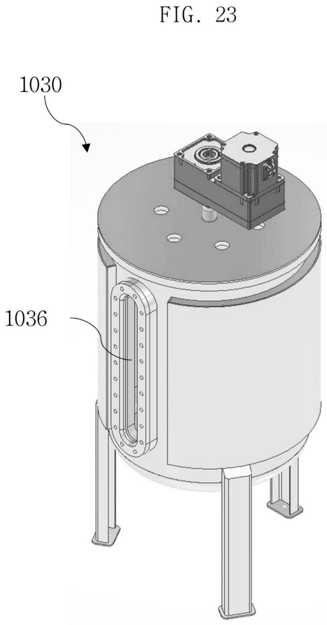

FIGS. 23 to 25 illustrate the monitoring module in the expandable producing module according to an embodiment of the present disclosure.

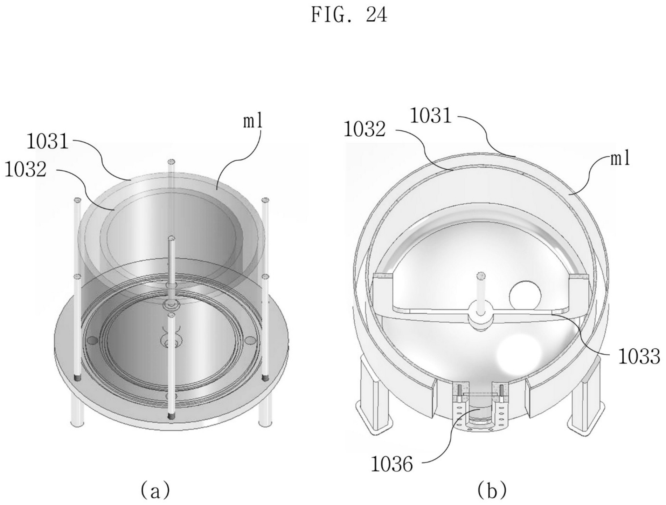

Referring to FIGS. 23 and 24, the monitoring module 1030 comprises an outer housing 1031 and an inner housing 1032. The monitoring module 1030 may have a dual jacket structure, similar to the monitoring module 1200 of FIG 7, with a multi-layer space (ml) between the housings. The inner housing 1032 may have an internal chamber for containing bioink.

The inner housing 1032 may be provided with an internal space capable of accommodating bioink. The multi-layer space (ml) may be equipped with a space through which fluid can circulate. The temperature of the bioink stored in the inner housing 1032 can be maintained at a constant level as the fluid circulates through the multi-layer space (ml) by the circulator 1020.

The monitoring module 1030 may include a window 1036 allowing users to visually identify the state of the bioink. The window 1036 may be formed in an elongated shape in a longitudinal direction, fomed outside the inner housing 1032, or may be a transparent polycarbonate material. This window allows visual inspection of the state of the bioink (e.g., the bioink's type and quantity) within the inner housing 1032.

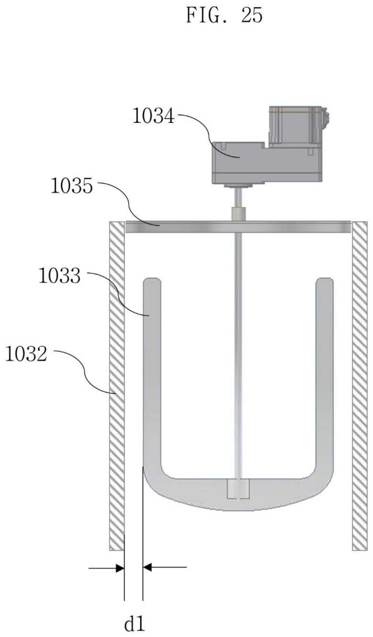

Referring to FIGS 24 and 25, the monitoring module 1030 may include a blade 1033, which may correspond to the blade 1230 described in FIG. 7.

The blade 1033 may be disposed within the inner housing 1032 by a predetermined distance d1. For example, the predetermined distance may be approximately 10 mm from the inner wall. If this space between the blade 1033 and the inner housing 1032 is formed within a predetermined distance, this facilitates efficient bioink agitation when the blade rotates.

A driving device 1034 powers the blade's rotation. At least one sensor may be located in either the driving device 1034 or the upper plate 1035. The monitoring module 1030 may monitor the temperature and pH of the bioink stored in the internal housing 1032 based on the value measured from the sensor.

The internal space of the monitoring module 1030 may be opened and closed by a separate device (e.g., a lifting device). The upper plate 1035 of the monitoring module 1030 can be raised and lowered by the separate lifting device to access the internal chamber.

A cleaning device may be installed within the inner housing 1032 of the monitoring module 1030 to maintain sanitary conditions. The cleaning device may be of various known types and clean the internal space.

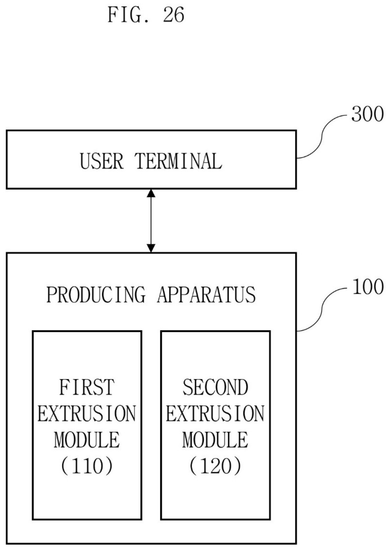

FIG. 26 depicts a method for controlling the extrusion modules based on user input according to an embodiment of the present disclosure. Referring to FIG. 26, the producing apparatus 100 receives user input from a user terminal 300 to control first and second extrusion modules 110, 120.

The user terminal 300 may obtain producing information for artificial meat or organs through an interface. This producing information may include specifications for a first and a second component constituting the artificial meat. The first component may be protein and the second component may be fat. The producing information may further specify the desired ratio of protein to fat.

The producing apparatus 100 may obtain artificial meat producing information obtained from an external device (e.g., user terminal) and control the first and second extrusion modules 110, 120 based on the obtained information. The first extrusion module 110 may be a module for extruding bioink (first bioink) related to a first component, and the second extrusion module 120 may be a module for extruding bioink (second bioink) related to a second component.

Based on this information, the producing apparatus 100 may determine extrusion parameters for each bioink type (e.g., the first bioink and the second bioink). These parameters may include extrusion start and end times, extrusion speed, and extrusion amount. The producing apparatus 100 determines at least one of a time when the first extrusion module 110 starts extruding the first bioink, a time when the extrusion ends, a speed of extrusion, and an amount of extrusion based on the producing information of the artificial meat. The producing apparatus 100 determines at least one of a time when the second extrusion module 120 starts extrusion of the second bioink, a time when the extrusion ends, a speed of extrusion, and an amount of extrusion based on the producing information of the artificial meat.

The producing apparatus 100 may monitor and control the status of the first bioink and the second bioink based on the producing information of the artificial meat. For example, the producing apparatus 100 monitors and adjusts bioink temperature and pH to meet predetermined conditions for each type of bioink.

The producing apparatus 100 may determine the pattern information about the artificial meat to be produced based on user input. Upon obtaining the pattern information, the producing apparatus 100 may determine extrusion parameters for both the first and second bioinks. These parameters are derived from the artificial meat pattern information. The producing apparatus 100 can simultaneously control multiple extrusion modules based on these determined parameters, thereby enabling the production of artificial meat with user-desired patterns

The configuration and operation of the first and second extrusion modules 110, 120 correspond to the descriptions provided in FIGS. 10 to 20.

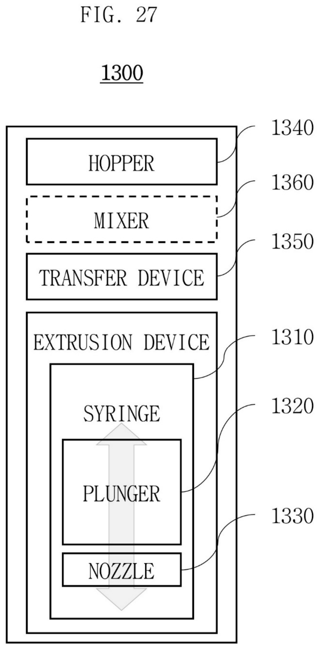

FIG. 27 illustrates an extrusion module according to another embodiment of the present disclosure. Referring to FIG. 27, the extrusion module 1300 may be a device configured to apply pressure to bioink and extrude it in the form of multiple fiber bundles. Since the configuration related to the extrusion device included in the drawing has already been described above, redundant descriptions will be omitted.

The present disclosure discloses a system for controlling the flow of a mixture. Here, the term "bioink" as used throughout this specification is not limited to a single specific component but may be interpreted as a mixture comprising various substances, such as high-viscosity fluids, biomaterials, edible materials, organic mixtures, inorganic mixtures, gels, powder formulations, and dough-like materials. This indicates that the technical scope of the present disclosure is not limited to a particular substance but encompasses technical features for efficiently transporting and controlling various types of mixtures under the category of bioink and the like.

Hereinafter, the term "bioink" will be used for the sake of consistency, but it shall be understood as encompassing the various types of materials included in the aforementioned mixtures.

At least a portion of the bioink stored in the hopper 1340 included in the extrusion module 1300 may be transferred into the inner space of the syringe 1310 by an external force.

The extrusion module 1300 may include a hopper 1340. The hopper 1340 may have an internal space capable of containing bioink. The hopper 1340 may be fluidly connected to at least a portion of a transfer device 1350. At least a portion of the bioink stored in the hopper 1340 may be introduced into the transfer device 1350 by external force or gravity.

The extrusion module 1300 may include a transfer device 1350. The transfer device 1350 may control the flow of bioink. The transfer device 1350 may be configured to transfer bioink contained in the hopper 1340 or the monitoring module 1200 to the syringe 1310 and to provide the driving force necessary for extrusion. The transfer device 1350 may provide a mechanism capable of efficiently and stably transferring fluids such as bioink, and may be designed to accommodate a wide range of material properties, including high-viscosity or low-viscosity bioinks. The transfer device 1350 may play a critical role in improving the accuracy and productivity of bioprinting or related manufacturing processes by enabling precise fluid delivery.

The transfer device 1350 may be implemented as one of various types of pumps, such as a piston pump that transfers fluid through a reciprocating motion. This configuration enables the fluid to be delivered at a consistent pressure and speed, minimizing potential losses or irregularities during the transfer process. The transfer device 1350 may be designed to stably transport bioink while maintaining its properties, thereby operating advantageously in precision manufacturing processes such as bioprinting.

The transfer device 1350 may include an extrusion device, which may comprise the syringe 1310, the plunger 1320, and the nozzle 1330 metioned above.

The extrusion module 1300 may include a mixer 1360. The mixer 1360 may be a device configured to create a homogeneous mixture by combining various materials, such as powder and water. The mixer 1360 may include rotating blades or stirring components that mix the materials internally, and may be designed to accommodate both high-viscosity and low-viscosity substances. The mixer 1360 may also be configured to preserve the physical or chemical properties of the materials during the mixing process.

In some cases, the extrusion module 1300 may further include a homogenizer or a grinder. The homogenizer may function to uniformly disperse high-viscosity mixtures or fine particles. The grinder may be used to break down solid materials to enhance mixing efficiency. The mixer 1360, the homogenizer, and the grinder are not essential components of the extrusion module 1300 and may be optionally included as needed.

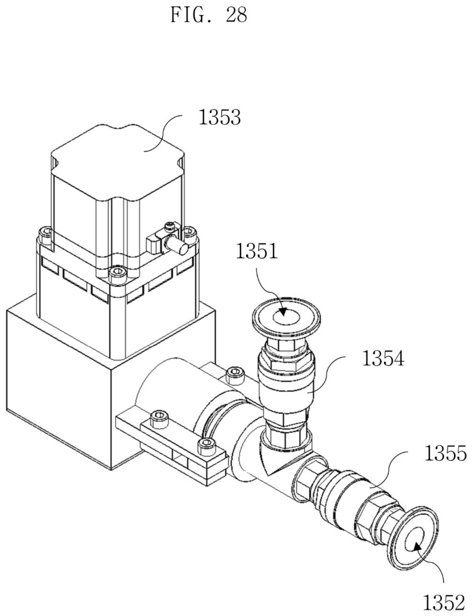

FIG. 28 illustrates a transfer device according to an embodiment of the present disclosure. Referring to FIG. 28, the transfer device 1350 may include an inlet 1351 through which bioink is introduced, an outlet 1352 through which the bioink is discharged, a motor 1353 that provides driving power to a piston, a first fluid channel 1354, a second fluid channel 1355, a piston 1356 that performs reciprocating motion within a cylinder, and a check valve 1357 positioned between the inlet and the outlet to control the flow of fluid.

The inlet 1351 may serve as an entry point through which bioink is introduced. The outlet 1352 may serve as an exit through which the bioink is discharged.

The inlet 1351 may be fluidly connected to the hopper 1340, and at least a portion of the bioink contained in the hopper 1340 may be supplied to the transfer device 1350 through the inlet 1351. The inlet 1351 may be formed in the direction of gravity, allowing at least a portion of the bioink stored in the hopper 1340 to flow into the inlet 1351 by gravitational force.

The outlet 1352 may be fluidly connected to the extrusion device, and at least a portion of the bioink present in the cylinder may be discharged through the outlet 1352 to the extrusion device by the reciprocating motion of the piston 1356. The outlet 1352 may be formed in a direction different from that of the inlet 1351 and may, for example, be formed in a direction perpendicular to the direction of the inlet 1351.

The motor 1353 may serve as a driving unit and provide the power necessary to operate the piston pump. The motor 1353 may generate rotational motion, which is converted into reciprocating motion of the piston pump to assist in the stable transfer of fluids such as bioink. Various known technologies may be applied to the motor 1353, and, for example, a brushless DC (BLDC) motor may be used. In such a case, the motor may offer high precision and energy efficiency, while also producing low noise and vibration, making it advantageous for transferring sensitive materials such as bioink. The motor 1353 may be operatively connected to a control system (e.g., at least one processor) and may operate in response to conditions such as fluid viscosity, flow rate, and pressure.

The first fluid channel 1354 and the second fluid channel 1355 may provide flow paths for the bioink. These channels may serve as conduits through which the bioink passes and may be formed in a cylindrical structure. The first fluid channel 1354 may be formed in connection with the inlet 1351, and the second fluid channel 1355 may be formed in connection with the outlet 1352.

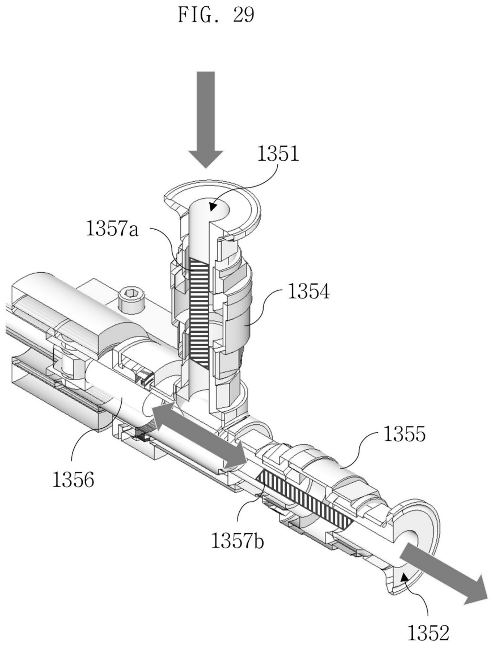

FIG. 29 illustrates the flow of fluid within a transfer device according to an embodiment of the present disclosure.

Referring to FIG. 29, when bioink is introduced into the inlet 1351 of the transfer device 1350, the bioink may pass through the first fluid channel 1354 and be delivered to the piston 1356, which performs a reciprocating motion within the cylinder. The bioink may then be moved by the piston 1356 into the second fluid channel 1355 and subsequently discharged through the outlet 1352.

The first fluid channel 1354 may include a first check valve 1357a, and the second fluid channel 1355 may include a second check valve 1357b. The check valves 1357a and 1357b may function to control the flow direction of the fluid and prevent backflow.

The first check valve 1357a may be positioned in a region adjacent to the inlet 1351 and may prevent backflow when bioink is delivered from the inlet 1351 through the first fluid channel 1354 to the piston 1356. The second check valve 1357b may be positioned in a region adjacent to the outlet 1352 and may ensure that, when the piston 1356 pushes the bioink into the second fluid channel 1355, the bioink does not flow backward but is discharged externally through the outlet 1352.

The transfer device 1350 may further include at least one processor, which may control the operation of the piston 1356 by executing at least one instruction stored in memory.

The processor may control the operating conditions of the piston 1356. Here, the operating conditions may relate to at least one of the reciprocating speed of the piston, reciprocating cycle, pressure, and the RPM value of the motor.

The processor may control the operation of the piston 1356 in response to the status of the extrusion device. Specifically, the processor may adjust the piston's operation based on the amount of bioink contained within the syringe 1310 of the extrusion device. For example, if the amount of bioink in the syringe 1310 falls below a predetermined threshold, the processor may control the piston 1356 to perform a reciprocating motion. Conversely, if the amount of bioink in the syringe 1310 exceeds a predetermined threshold, the processor may control the piston 1356 to stop its operation.

The processor may control the operation of the piston 1356 based on the state of the bioink. Specifically, the processor may adjust the piston's operation under different conditions depending on the viscosity of the bioink. The processor may classify the bioink's state as one of low viscosity, medium viscosity, or high viscosity, and control the piston 1356 accordingly. For example, if the bioink is determined to be of low viscosity, the processor may operate the piston at a first speed; if the bioink is of medium viscosity, the piston may be controlled at a second speed; and if the bioink is of high viscosity, the piston may be controlled at a third speed.

For example, the first speed may be faster than the second speed, and the second speed may be faster than the third speed. More specifically, when the bioink is of low viscosity, the processor may operate the piston at a high reciprocating speed to provide a high flow rate. When the bioink is of medium viscosity, the piston may be operated at a medium speed to ensure a uniform flow. When the bioink is of high viscosity, the processor may reduce the reciprocating speed of the piston to allow the bioink to move stably at a low speed.

In another example, the first speed may be slower than the second speed, and the second speed may be slower than the third speed.

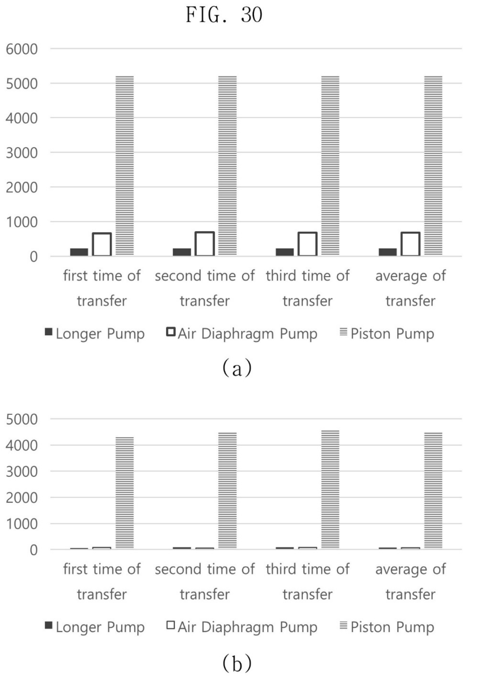

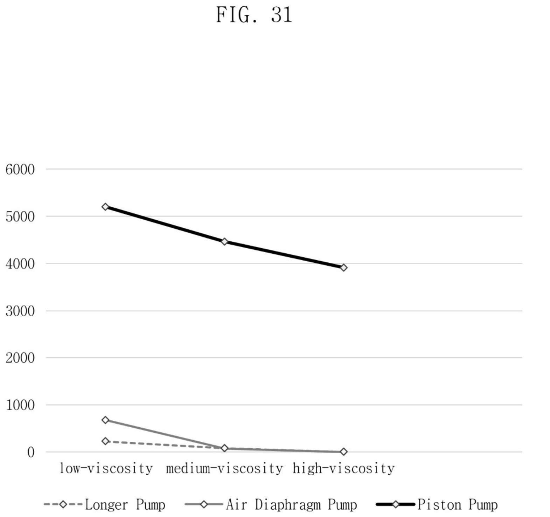

FIGS. 30 and 31 are diagrams illustrating the transfer efficiency when using the transfer device according to an embodiment of the present disclosure.

An experiment was conducted to evaluate the efficiency of transferring bioink with various viscosities using the transfer device 1350 of the present embodiment and to verify its performance in comparison with conventionally known types of pumps, such as a Longer Pump and an Air Diaphragm Pump.

For this experiment, the bioink was categorized into low, medium, and high viscosity types based on viscosity.

Specifically:
The low-viscosity bioink was prepared with a composition of 2% alginate and 98% water.

The medium-viscosity bioink was composed of 2% alginate, 1% methylcellulose (M.C), 2% fish collagen, 15% palm oil, and 80% water.

The high-viscosity bioink was prepared with 2% alginate, 1.5% methylcellulose (M.C), 1% ISP, 0.5% salt, 0.5% sugar, 36% sunflower oil, and 58.5% water.

In this experiment, a Longer Pump, an Air Diaphragm Pump, and the transfer device 1350 were used. The initial weight of an empty container was measured, and then bioink was transferred using each pump for a duration of three minutes. After the transfer, the weight of the container was measured again to calculate the weight of the transferred bioink. The experiment was repeated three times for each pump, and the average transferred volume and efficiency were compared based on the repeated trials.

FIG. 30(a) shows the results of comparing the transfer efficiency for low-viscosity bioink. Referring to FIG. 30(a), the average transferred volume was 222.3 g for the Longer Pump, 675.0 g for the Air Diaphragm Pump, and 5200.0 g for the transfer device 1350. As a result, the transfer device 1350 of the present invention demonstrated significantly higher efficiency-approximately 23 times greater than the Longer Pump and approximately 7.7 times greater than the Air Diaphragm Pump-when transferring low-viscosity bioink.

FIG. 30(b) shows the results of comparing the transfer efficiency for medium-viscosity bioink. Referring to FIG. 30(b), the average transferred volume was 77.3 g for the Longer Pump, 73.5 g for the Air Diaphragm Pump, and 4466.0 g for the transfer device 1350. While the Longer Pump and the Air Diaphragm Pump showed similar levels of efficiency, the transfer device 1350 demonstrated significantly superior performance, achieving approximately 60 times greater transfer efficiency in handling medium-viscosity bioink.

Meanwhile, in terms of transferring high-viscosity bioink, both the Longer Pump and the Air Diaphragm Pump were unable to transfer the bioink effectively. In contrast, the transfer device 1350 of the present invention successfully transferred the high-viscosity bioink, achieving an average transferred volume of approximately 3916.7 g.

FIG. 31 is a graph comparing the transfer efficiency of each device by viscosity category. Referring to FIG. 31, it can be observed that the transfer efficiency of all devices decreases as the viscosity increases. Specifically, for low-viscosity bioink, the transfer device 1350 exhibited the highest efficiency, followed by the Air Diaphragm Pump and then the Longer Pump. For medium and higher viscosities, the transfer device 1350 consistently demonstrated the highest efficiency, while there was little difference in performance between the Air Diaphragm Pump and the Longer Pump. Notably, for high-viscosity bioink, neither the Air Diaphragm Pump nor the Longer Pump was able to operate, whereas only the transfer device 1350 was capable of functioning.

These experimental results demonstrate that the transfer device 1350 of the present invention is the most effective solution for efficiently transferring bioink across a wide range of viscosities.

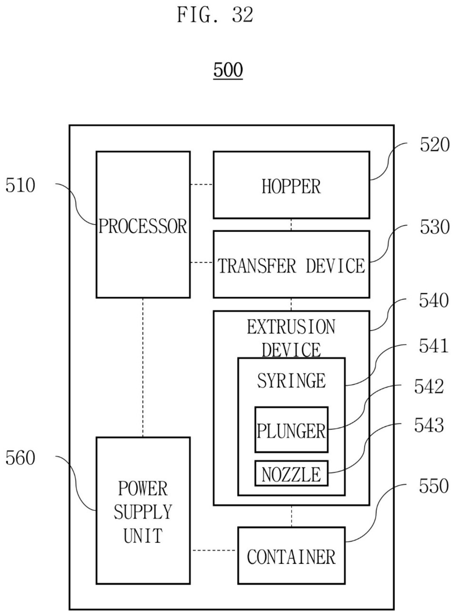

FIG. 32 illustrates a producing apparatus according to another embodiment of the present disclosure. Referring to FIG. 32, the producing apparatus 500 may include a processor 510, a hopper 520, a transfer device 530, an extrusion device 540, a container 550, and a power supply unit 560. The producing apparatus 500 may be configured as a mobile structure and may further include a driving unit for movement.

The processor 510 functions as the control unit of the producing apparatus 500 and may control the operation of the transfer device 530 and the extrusion device 540 in real time. The processor 510 may analyze process conditions such as the viscosity of the bioink, extrusion speed, and flow rate, and control the system to maintain optimal operating conditions. The processor 510 may also precisely adjust the operation of the apparatus based on user-defined parameters.

The hopper 520 may serve to store and supply bioink used in the manufacturing process. It may be designed to maintain the bioink in a stable condition and be capable of accommodating both high-viscosity and low-viscosity bioinks.

The transfer device 530 may function to stably transfer bioink from the hopper 520 to the extrusion device 540. It may employ a piston-based design that maintains a consistent flow rate and pressure regardless of the viscosity characteristics of the bioink and may include check valves to prevent backflow of the fluid. Since this structure is the same as that described above with reference to FIGS. 5 through 8, a redundant explanation is omitted.

The extrusion device 540 may serve to extrude the bioink supplied by the transfer device 530. It is designed to discharge the bioink in the desired form or position for its final application and provides precise extrusion control to ensure accurate and consistent output.

The container 550 may serve as a space for storing or temporarily holding the bioink extruded from the extrusion device 540. It may be made of a material that prevents damage or degradation of the bioink and may be designed to facilitate further processing or storage after transfer, depending on the intended use.

Since the producing apparatus 500 may be designed as a mobile structure, it may include a power supply unit 560. The power supply unit 560 can provide stable electrical power to all components of the apparatus, enabling the producing apparatus 500 to operate not only in fixed locations but also in various working environments.

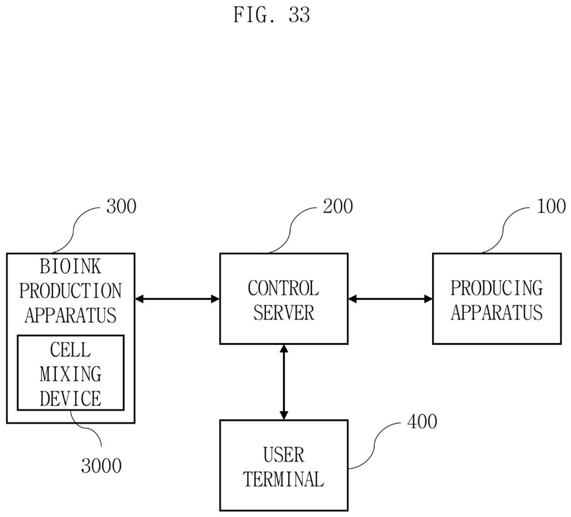

FIG. 33 illustrates a cell mixing device according to an embodiment of the present disclosure. Referring to FIG. 33, an bioink production apparatus 300 according to the embodiment may include a cell mixing device 3000.

The cell mixing device 3000 is used for obtaining extrusion material by uniformly mixing cells with at least one other substance, thereby optimizing the quality of the extrusion material. The cell mixing device 3000 enables uniform distribution of cells within the extrusion material and may also adjust the physical and/or chemical properties of the composition to facilitate the formation of a specific tissue structure. During the mixing process, the cell mixing device 3000 may assist in regulating viscosity and flowability to ensure a smooth extrusion process, and it may precisely control the mixing speed and method to prevent physical damage to the cells.

The cell mixing device 3000 may be provided within the producing apparatus 100. Through the cell mixing device 3000 included in the producing apparatus 100, the extrusion material may be prepared, and the completed material may subsequently be processed into cultured meat.

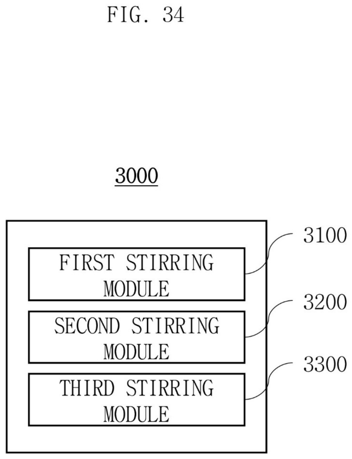

FIG. 34 illustrates the configuration of a cell mixing device according to an embodiment of the present disclosure. Referring to FIG. 34, the cell mixing device 3000 may include a first stirring module 3100, a second stirring module 3200, and a third stirring module 3300.

The first stirring module 3100 and the second stirring module 3200 may serve as primary mixing modules, while the third stirring module 3300 may function as a secondary mixing module. The primary mixing modules may be configured to produce pre-extrusion material, and the secondary mixing module may be configured to produce the final extrusion material.

More specifically, the primary mixing modules may mix cells with a predefined first material to obtain the pre-extrusion material. The secondary mixing module may then mix the pre-extrusion material with a predefined second material to produce the final extrusion material suitable for further processing.

Although the drawings illustrate the cell mixing device 3000 as including three stirring modules, the configuration is not limited thereto. The cell mixing device 3000 may include a primary stirring module (e.g., the first stirring module) and a secondary stirring module (e.g., the third stirring module). Alternatively, the cell mixing device 3000 may include three or more primary stirring modules and two or more secondary stirring modules.

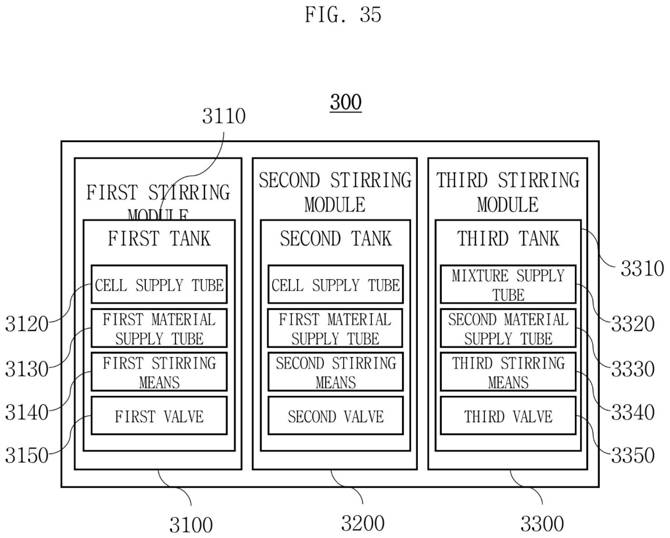

FIG. 35 illustrates the detailed configuration of a stirring module according to an embodiment of the present disclosure. Referring to FIG. 35, the first stirring module 3100 may include a first tank 3110, a cell supply tube 3120, a first material supply tube 3130, first stirring means 3140, and a first valve 3150.

The first stirring module 3100 may include a first tank 3110 having an internal space for containing materials. The first tank 3110 may include: a cell supply tube 3120 for introducing cells into the interior of the tank; a first material supply tube 3130 for introducing a first material into the tank; a first stirring means 3140 for performing a mixing operation of the materials; and a first valve 3150 for controlling the discharge of the materials contained in the tank.

The cell supply tube 3120 serves as a passage for delivering cultured cells into the interior of the first tank 3110 and may be designed to maintain a constant flow rate while preventing damage to the cells. The first material supply tube 3130 may serve to inject various substances into the first tank 3110, including bio-materials, culture media, PBS solution (phosphate-buffered saline), physiological saline, solutions exhibiting non-Newtonian fluid properties, alginate, or other additives. The diameter and pressure of the tube may be adjusted according to the viscosity and flow characteristics of the materials being introduced.

The first stirring means 3140 functions to uniformly mix the introduced cells and the first material. It may be designed to allow adjustments to stirring speed, rotation method (e.g., rotary blades), and stirring intensity, thereby enabling efficient and homogeneous mixing of the components.

The first valve 3150 serves to control the opening and closing during the discharge of the mixed material. By adjusting the discharge rate, it enables consistent flow and the delivery of a homogeneous mixture to subsequent processes.

The third stirring module 3300 may include a third tank 3310 having an internal space for containing materials. The third tank 3310 may include: a mixture supply tube 3320 for introducing materials mixed in the first stirring module 3100 or the second stirring module 3200 into the tank; a second material supply tube 3330 for introducing a second material into the tank; third stirring means 3340 for performing a mixing operation of the materials; and a third valve 3350 for controlling the discharge of the materials contained in the tank.

As described above, the mixture supply tube 3320 serves as a passage for delivering materials (e.g., a mixture) mixed in the first stirring module 3100 or the second stirring module 3200 into the interior of the third tank 3310 and may be designed to maintain a constant flow rate. The second material supply tube 3330 may serve to inject into the third tank 3310 various substances that can be mixed with the aforementioned mixture, such as bio-materials, culture media, PBS solution (phosphate-buffered saline), physiological saline, solutions exhibiting non-Newtonian fluid properties, alginate, or other additives. The second material may be the same as or different from the first material.

The third stirring means 3340 performs the function of uniformly mixing the introduced mixture and the second material, and may be of a different type than the stirring means included in the primary stirring modules.

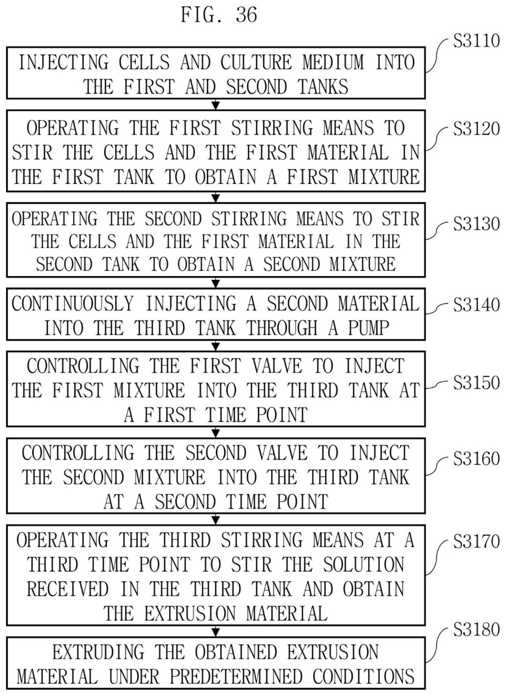

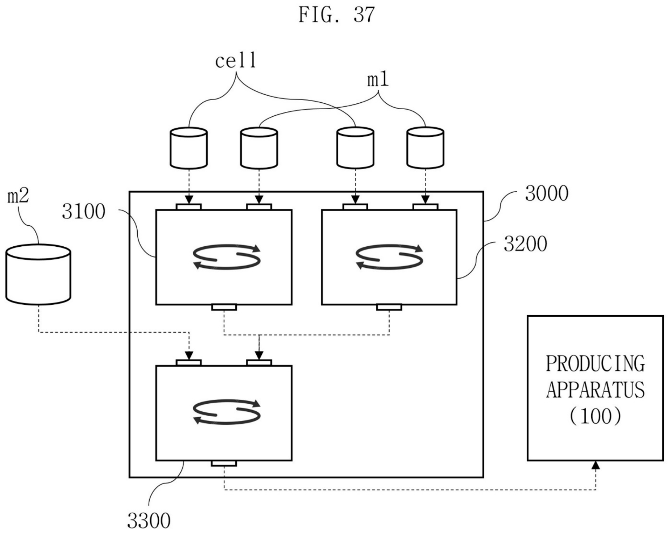

FIG. 36 and FIG.37 illustrate the operation of a cell mixing device according to an embodiment.

Referring to FIG. 36, the cell mixing device 3000 according to the embodiment may operate through the following steps:
Step S3110: Injecting cells and culture medium (or PBS solution (phosphate-buffered saline), physiological saline, a non-Newtonian fluid, alginate, or other additives) into the first and second tanks.
Step S3120: Operating the first stirring means to stir the cells and the first material in the first tank to obtain a first mixture.
Step S3130: Operating the second stirring means to stir the cells and the first material in the second tank to obtain a second mixture.
Step S3140: Continuously injecting a second material into the third tank through a pump.
Step S3150: Controlling the first valve to inject the first mixture into the third tank at a first time point.
Step S3160: Controlling the second valve to inject the second mixture into the third tank at a second time point.
Step S3170: Operating the third stirring means at a third time point to stir the solution received in the third tank and obtain the extrusion material.
Step S3180: Extruding the obtained extrusion material under predetermined conditions.

Through this process, the cell mixing device 3000 can produce an extrusion material by mixing cells with predefined materials via a plurality of stirring modules.

Referring to FIG. 37, the cell mixing device 3000 can control the first mixing module 3100, second mixing module 3200, and third mixing module 3300 in parallel. The device is configured that the mixtures prepared in the first mixing module 3100 and second mixing module 3200 can be sequentially introduced into the third mixing module 3300.

The cell mixing device 3000 may first introduce the mixture contained in the first stirring module 3100 into the third stirring module 3300, and after the operation is completed, may sequentially introduce the mixture contained in the second stirring module 3200 into the third stirring module 3300. The cell mixing device 3000 may sequentially introduce the mixture contained in the first stirring module 3100 and the mixture contained in the second stirring module 3200 into the third stirring module 3300 at different times.

The cell mixing device 3000 may control the first valve 3150 at a first time point to introduce the first mixture into the third tank 3310 and may operate the third stirring means 3340 at a third time point to perform a mixing operation of the material contained in the third tank 3310. The third time point is after the first time point and may be a time point at which it is confirmed that the first mixture has been completely introduced into the third tank 3310.

For example, the first material may be a culture medium (or a PBS solution (phosphate-buffered saline), physiological saline, a solution exhibiting non-Newtonian fluid properties, alginate, or other additives), and the first mixture may be a material in which the cells and the first material are mixed. The second material may be an alginate solution, and the second mixture may be a material in which the first mixture and the alginate solution are mixed.

The first stirring unit 3140, the second stirring unit 3240 and the third stirring unit 3340 may be implemented in various forms depending on the physical properties of the materials to be mixed and the required level of mixing homogeneity.

For example, the stirring unit may be based on vibration, operating by inducing flow within the mixture using oscillations. In another example, it may be an impeller-based stirring mechanism, which forms a strong fluid flow using rotating impeller blades. This method is effective not only for culture media but also for mixing high-viscosity polymer materials such as alginate solutions. The shape of the impeller may be one of various well-known types, including the Rushton type, Marine type, Turbine type, or Helical Impeller type. In particular, when implemented as a helical impeller, it can be advantageous for mixing high-viscosity substances due to the spiral structure, which induces a uniform flow and is more favorable for improving cell viability.

In another example, the stirring unit may be a static mixer, which enables multi-stage cross-mixing of fluids through fixed internal pattern structures. Alternatively, the stirring unit may function by shaking the housing (tank) itself. For example, mixing methods such as linear reciprocating motion, rotary vibration, or gyroscopic mixing may be applied to shake the housing.

At least one of the stirring units among the first stirring unit 3140, the second stirring unit 3240 and the third stirring unit 3340 may employ a mixing method using a 3-way stopcock valve. The 3-way stopcock valve may include a first inlet, a second inlet, an internal mixing chamber, and an outlet. In this case, cells and the first material may be introduced through the first and second inlets, respectively, and the introduced substances may be naturally mixed within the internal mixing chamber, with the resulting mixture discharged through the outlet.

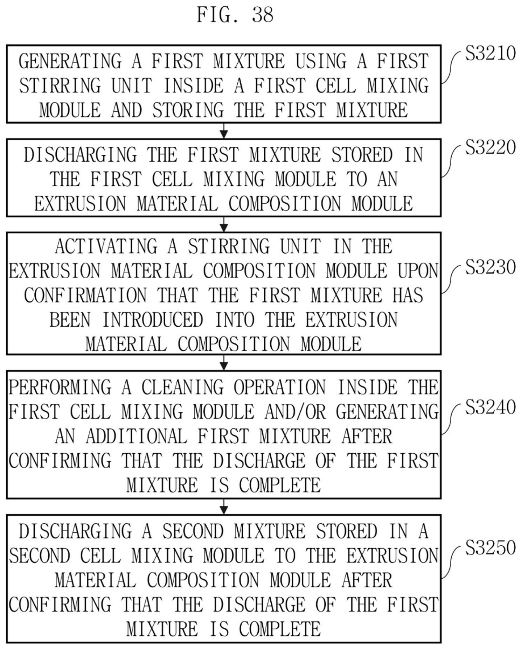

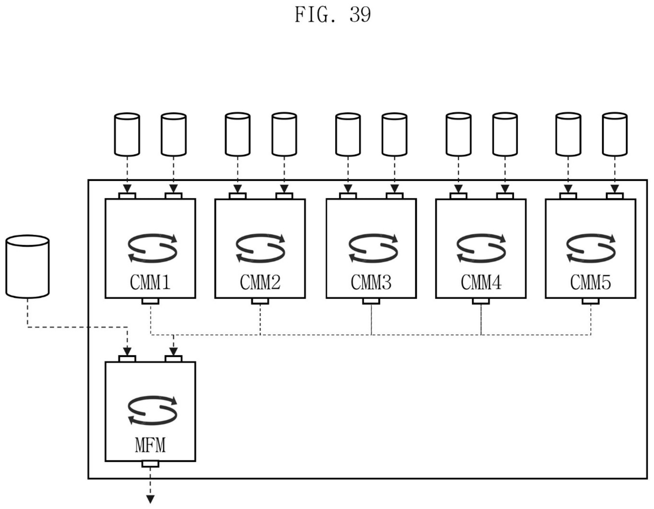

FIGS. 38 and 39 are diagrams illustrating a method of operating a plurality of cell mixing modules and an extrusion material composition module according to an embodiment.

Referring to FIG. 38, the cell mixing device 3000 according to an embodiment may operate through the following steps:
Step S3210: generating a first mixture using a first stirring unit inside a first cell mixing module and storing the first mixture;
Step S3220: discharging the first mixture stored in the first cell mixing module to an extrusion material composition module;
Step S3230: activating a stirring unit in the extrusion material composition module upon confirmation that the first mixture has been introduced into the extrusion material composition module;
Step S3240: performing a cleaning operation inside the first cell mixing module and/or generating an additional first mixture after confirming that the discharge of the first mixture is complete; and
Step S3250: discharging a second mixture stored in a second cell mixing module to the extrusion material composition module after confirming that the discharge of the first mixture is complete.

Referring to FIG. 38 and FIG. 39, the first cell mixing module(CMM1) through fifth cell mixing module(CMM5) may correspond to the above-described primary stirring modules, and the material forming module(MFM) may correspond to the above-described secondary stirring module.

The cell mixing device 3000 may control a plurality of cell mixing modules (CMMs) in parallel and may also control each cell mixing module (CMM) sequentially. For example, the cell mixing device 3000 may first introduce the mixture contained in the first cell mixing module(CMM1) into the material forming module(MFM). Upon completion of the stirring operation in the material forming module(MFM), the cell mixing device 3000 may sequentially introduce the mixture contained in the second cell mixing module(CMM2) into the material forming module(MFM).

The cell mixing device 3000 may perform a cleaning operation inside the first cell mixing module(CMM1) once it is confirmed that the first mixture has been discharged from the first cell mixing module(CMM1). The first cell mixing module(CMM1) may include a waste discharge unit, and wastewater generated during the internal cleaning may be discharged to the outside through the waste discharge unit.

For example, the cell mixing device 3000 may monitor cells introduced into the first cell mixing module(CMM1) and determine whether to perform an internal cleaning operation of the first cell mixing module(CMM1) based on the type of cells being introduced. For instance, if it is confirmed that first cells have been introduced into the first cell mixing module(CMM1) and the mixture related to the first cells has been discharged, and if it is subsequently determined that the newly introduced cells differ from the first cells, the cell mixing device 3000 may perform an internal cleaning operation of the first cell mixing module (CMM1).

In another example, the cell mixing device 3000 may include at least one of a residual cell detection sensor, a contaminant detection sensor, a conductivity sensor, and a pH sensor, and may determine whether internal cleaning of the first cell mixing module(CMM1) is required based on the values measured by the sensor(s).

The cell mixing device 3000 may perform an operation of discharging the remaining residue from the inside of the first cell mixing module(CMM1) when it is confirmed that the first mixture has been discharged from the first cell mixing module(CMM1). For example, the residue discharge operation may involve scraping the inside of the housing using a tool provided in the first cell mixing module(CMM1). More specifically, the residue discharge operation may be performed by injecting high-pressure air or cleaning liquid, or by using a rotary scraper.

The cell mixing device 3000 may perform an operation of generating an additional first mixture by operating the first stirring means when it is confirmed that the first mixture has been completely discharged from the first cell mixing module(CMM1). The operation of generating the additional mixture may be carried out in a pre-mixing manner, in which, while the first mixture is being discharged, the second mixture is pre-stirred in the second cell mixing module(CMM2), so that a continuous process flow can be maintained.

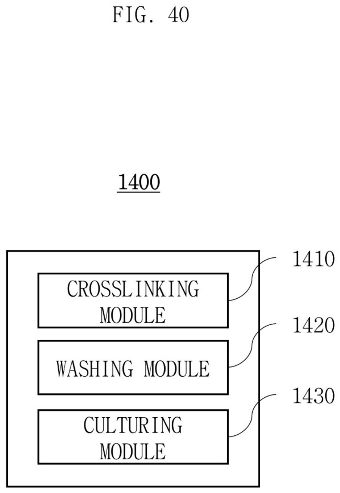

FIG. 40 illustrates a post-processing module according to an embodiment. Referring to FIG. 40, the post-processing module 1400 according to an embodiment may include a crosslinking module(gelation module) 1410, a washing module 1420, and a culturing module 1430.

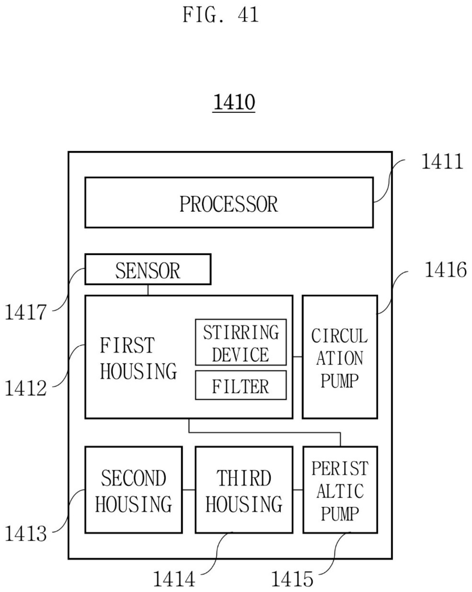

FIG. 41 illustrates the configuration of a crosslinking module according to an embodiment. As described above, the crosslinking module 1410 may serve to perform a crosslinking reaction to ensure the structural stability of the extruded material and facilitate final tissue formation. The crosslinking module 1410 may be implemented using hydrogel-based materials such as alginate and may crosslink the extruded material by reacting it with a specific ion solution (e.g., a CaCl₂ solution) or other compound solutions (e.g., iron ion solution, copper ion solution, magnesium ion solution, polylysine) or enzymes such as transglutaminase.

Referring to FIG. 41, the crosslinking module 1410 may include a processor 1411, a first housing 1412, a second housing 1413, a third housing 1414, a peristaltic pump 1415, a circulation pump 1416, and a sensor 1417.

The processor 1411 controls, in real time, the operation of components included in the crosslinking module 1410 and can control the circulation system. The processor 1411 collects data from the sensor 1417 to form a feedback loop so that the crosslinking reaction can be performed under optimal conditions and dynamically adjusts the flow of the solution by controlling the operation of the pumps 1415 and 1416 according to the conditions.

The first housing 1412 may include an internal space capable of containing a specific ionic solution (e.g., CaCl₂ solution). A crosslinking reaction between the extruded material and the ionic solution may occur within the internal space.

The internal space of the first housing 1412 may include a stirring device. The stirring device may induce the flow of the ionic solution contained within the first housing 1412 and maintain a uniform distribution of the solution. Stirring method may employ various known means, such as a rotary impeller, a magnetic stirrer, or an ultrasonic stirrer.

The internal space of the first housing 1412 may include a filter. The filter may function to prevent the extruded material from being drawn into the pumps 1415 and 1416. For example, the filter may be implemented as a mesh filter having a specific pore size, a porous membrane, or a cyclone filter.

The second housing 1413 may include an internal space in which unused ionic solution can be stored. At least a portion of the ionic solution contained in the second housing 1413 may be supplied to the first housing 1412 according to predetermined criteria. The second housing 1413 may be configured to detect the concentration of the solution in real time in conjunction with the sensor 1417, and to replenish fresh ionic solution when the concentration falls below a threshold. This operation may be controlled by the processor 1411.

The third housing 1414 may include an internal space in which purified water can be stored. As the crosslinking reaction progresses, the solution within the first housing 1412 is gradually consumed. When the amount of solution decreases below a predetermined threshold, at least a portion of the purified water stored in the third housing 1414 may be automatically supplied to the first housing 1412. This operation may be controlled by the processor 1411.

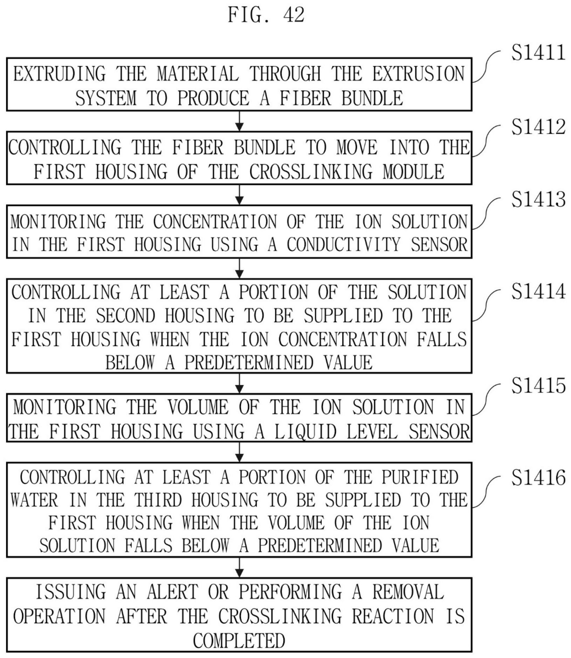

FIG. 42 illustrates a method by which the processor 1411 controls the operation of the crosslinking module according to one embodiment.

Referring to FIG. 42, the processor 1411 may perform the following operations:
Step 1411: processing the extruded material through an extrusion system to produce a fiber bundle;
Step 1412: controlling the fiber bundle to move into the first housing 1412 of the crosslinking module;
Step 1413: monitoring the concentration of the ion solution (e.g., CaCl₂) within the first housing 1412 using a conductivity sensor;
Step 1414: controlling at least a portion of the solution in the second housing 1413 to be supplied to the first housing 1412 when the concentration of the ion solution falls below a predetermined value;
Step 1415: monitoring the volume of the ion solution in the first housing 1412 using a liquid level sensor (S1415); and
Step 1416: controlling at least a portion of the purified water in the third housing 1414 to be supplied to the first housing 1412 when the volume of the ion solution falls below a predetermined value .

The processor 1411 may control the fiber bundle to be positioned in a specific region within the first housing 1412. The internal space of the first housing 1412 may be divided into a plurality of regions, and the processor 1411 may determine one of the plurality of regions as a target region based on a predetermined criterion. The processor 1411 may control the extruded fiber bundle to be located in the target region. For example, the target region may be one of the plurality of regions in which a fiber bundle is not currently located.

The processor 1411 may measure the ion concentration of the solution in the first housing 1412 using a conductivity sensor and may control at least a portion of the solution stored in the second housing 1413 to be supplied into the first housing 1412 based on the measurement. The processor 1411 may determine whether the ion concentration in the first housing 1412 is within a suitable range for maintaining the crosslinking reaction using the conductivity sensor, and if it is determined, based on the sensor value, that the ion concentration is diluted or needs replenishment, the processor 1411 may control at least a portion of the solution in the second housing 1413 to be supplied into the first housing 1412.

The processor 1411 may control the system to adjust the crosslinking reaction time or perform an additional stirring operation based on the ion concentration in the first housing 1412.

The processor 1411 may monitor the remaining amount of the ion solution in the first housing 1412 using a level sensor or a weight sensor, and if it is determined that the ion solution has decreased below a predetermined threshold, the processor 1411 may perform a step of automatically supplying purified water from the third housing 1414. If the processor 1411 determines that the ion concentration in the first housing 1412 has been diluted beyond the reference value due to the supply of purified water from the third housing 1414, it may additionally supply the ion solution from the second housing 1413 into the first housing 1412.

Although not shown in the drawings, the crosslinking module 1410 may further include a fourth housing. The solution contained in the internal space of the fourth housing may be the same as or similar to the solution contained in the internal space of the first housing 1412. The processor 1411 may control at least a portion of the solution in the fourth housing to be supplied into the first housing if the concentration or volume of the solution in the first housing 1412 does not satisfy a predetermined criterion based on sensor values.

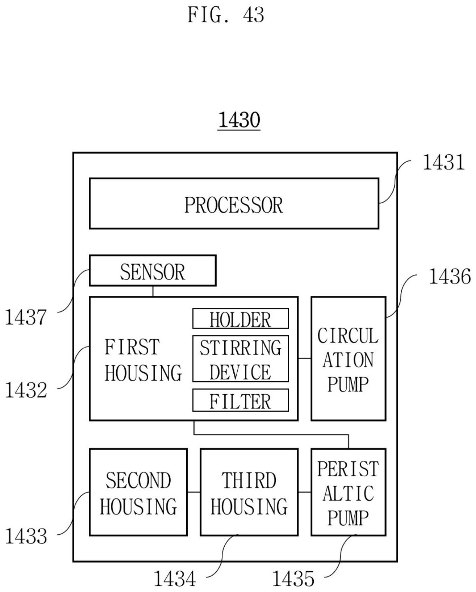

FIG. 43 illustrates a diagram for explaining the configuration of a culturing module according to an embodiment. The culturing module 1430 may serve to culture the extruded material that has undergone the crosslinking reaction for a predetermined period of time.

Referring to FIG. 43, the culturing module 1430 may include a processor 1431, a first housing 1432, a second housing 1433, a third housing 1434, a peristaltic pump 1435, a circulation pump 1436, and a sensor 1437.

The processor 1431 may control in real time the operation of all components included in the culturing module 1430 and perform operations for automatically adjusting the culturing environment. The processor 1431 may collect data from the sensor 1437 to monitor the temperature, pH, and nutrient concentration of the culture medium and form a feedback loop. If necessary, it may automatically perform operations for replenishing or replacing the culture medium. The processor 1431 may also control the operation of the pumps 1435 and 1436 to optimize the flow of the culture medium and support uniform culturing of the extruded material under consistent conditions.

The first housing 1432 may provide an internal space in which culturing is performed, and a culture medium mixture may be stored in the internal space. The extruded material may remain in the first housing 1432 for a predetermined period of time, during which the cells may grow and undergo tissue formation.

The interior of the first housing 1432 may be controlled to maintain constant temperature, pH, dissolved oxygen level, and nutrient concentration. The first housing 1432 may include a stirring device, which may be used to ensure uniform distribution of the culture medium mixture. The first housing 1432 may also include a filter to prevent the cultured extruded material from being drawn in by the pumps 1435 and 1436.

The first housing 1432 may include a holder. The holder may function to control the extruded material so that it does not move outside a specific area among the plurality of regions within the internal space of the first housing 1432.

The second housing 1433 may include an internal space for storing fresh culture medium mixture, which may be used to replenish the culture medium mixture consumed during the culturing process. The internal environment of the culture medium mixture within the second housing 1433 may be controlled to maintain a predetermined nutrient composition, pH, and temperature.

The second housing 1433 may contain a first type of culture medium component, and the third housing 1434 may contain a second type of culture medium component.

Alternatively, the third housing 1434 may include an internal space for storing purified water, and when the fluid level in the first housing 1432 decreases, the purified water may be supplemented. For example, during the culturing process in the first housing 1432, the culture medium mixture may evaporate or be consumed, resulting in changes in concentration or remaining volume, and purified water may be additionally supplied to maintain consistency.

The above-mentioned sensors 1417 and 1437 may include a plurality of sensors such as pH sensor, temperature sensor, oxygen sensor, conductivity sensor, level sensor, and weight sensor.

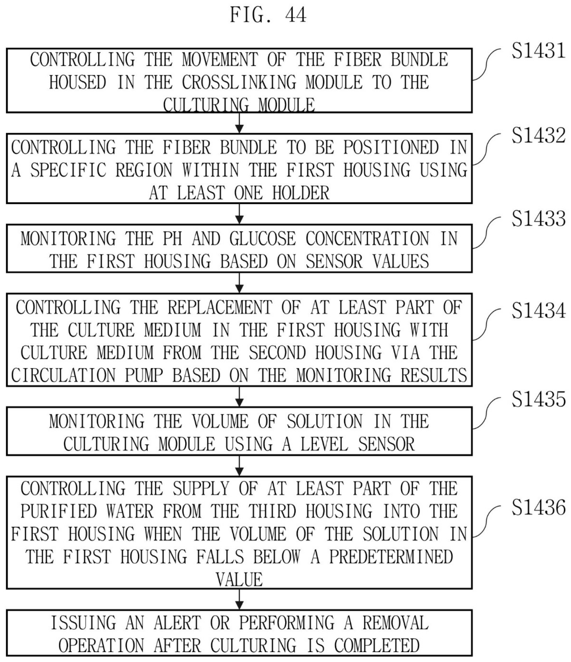

FIG. 44 is a diagram illustrating a method by which the processor controls the operation of the culturing module according to an embodiment.

Referring to FIG. 44, the processor 1431 may perform the following operations:
Step 1431: controlling the movement of the fiber bundle housed in the crosslinking module to the culturing module;
Step 1432: controlling the fiber bundle to be positioned in a specific region within the first housing using at least one holder;
Step 1433: monitoring the pH and glucose concentration in the first housing based on sensor values;
Step 1434: of controlling the replacement of at least part of the culture medium in the first housing with culture medium from the second housing via the circulation pump based on the monitoring results;
Step 1435: monitoring the volume of solution in the culturing module using a level sensor; and
Step 1436: controlling the supply of at least part of the purified water from the third housing into the first housing when the volume of the solution in the first housing falls below a predetermined value.

The processor 1431 may control the fiber bundle to be positioned in a specific region within the first housing 1432 using at least one holder. The internal space of the first housing 1432 may be divided into a plurality of regions, and the processor 1431 may determine one of the plurality of regions as a target region based on predetermined criteria. The processor 1431 may control the fiber bundle to be cultured within the target region by using the holder. As such, the holder may serve the function of fixing the fiber bundle so that it does not deviate from the specific region within the first housing 1432.

The processor 1431 may monitor the pH, glucose concentration, and other parameters within the first housing 1432 via the sensor, and based on this monitoring, control at least a portion of the culture medium mixture in the second housing 1433 to be supplied into the first housing 1432. The processor 1431 may determine, using the sensor, whether the culture medium mixture in the first housing 1432 is suitable for the culturing environment, and if it determines that replacement or supplementation is necessary, it may control at least a portion of the culture medium mixture in the second housing 1433 to be supplied into the first housing 1432.

The processor 1431 may perform an operation of adjusting the culturing time based on the monitoring results of the culture medium mixture within the first housing 1432.

The processor 1431 may monitor the remaining amount of the culture medium mixture in the first housing 1432 using a level sensor or a weight sensor, and if it determines that the amount has decreased below a predetermined threshold, it may automatically supply purified water from the third housing 1434.

If the processor 1431 determines that the culture medium mixture in the first housing 1432 has been diluted beyond a reference concentration due to the supply of purified water from the third housing 1434, it may additionally control the supply of culture medium mixture from the second housing 1433 into the first housing 1432.

The processors 1411 and 1431 may individually record the time points at which the extruded material is placed in specific regions of the first housings 1412 and 1432, respectively. The processors 1411 and 1431 may divide the interior of the housings into multiple crosslinking regions or culturing regions and may measure and monitor the processing status of the material located in each region.

As the processors 1411 and 1431 record the input time of each material, they may control operations such that the material is removed or subjected to additional processing after a certain period of time has elapsed. For example, the appropriate crosslinking and culturing durations may vary depending on the type of material being crosslinked or cultured (e.g., adipose fibers, protein fibers, etc.). The processors 1411 and 1431 may set individual timers based on the optimal culturing or crosslinking time for each material type, and if a specific material exceeds the preset time, an alert may be provided or an automatic operation for material removal may be performed.

The processors 1411 and 1431 may perform operations to ensure that materials exceeding the designated crosslinking or culturing time are discharged outside the housings 1412 and 1432 through a removal module (e.g., an automated transport device, discharge valve, or robotic arm). The processors 1411 and 1431 may automatically detect materials that have remained at a specific location within the housings 1412 and 1432 for a predetermined duration and control operations to discharge them accordingly.

The processors 1411 and 1431 may provide an alert to the user when the culturing and/or crosslinking time of the material is completed, and may configure an interface to allow the user to manually verify and remove the material.

Meanwhile, the crosslinking module 1410 and the culturing module 1430 may include a waste discharge unit, through which waste generated during the circulation of the solution can be discharged to the outside.

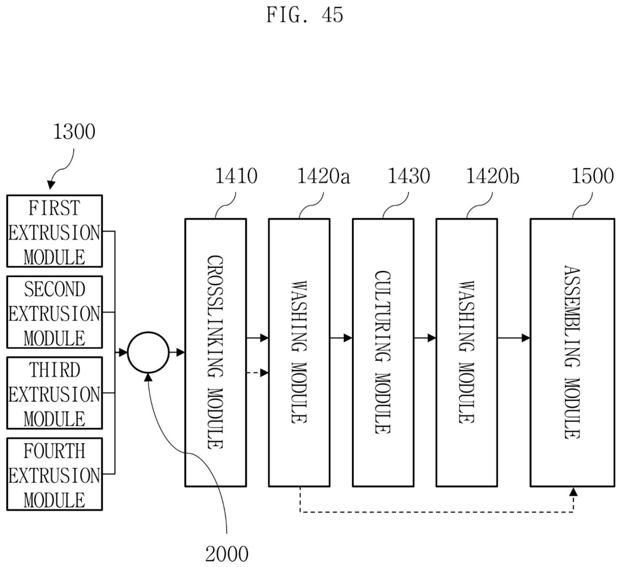

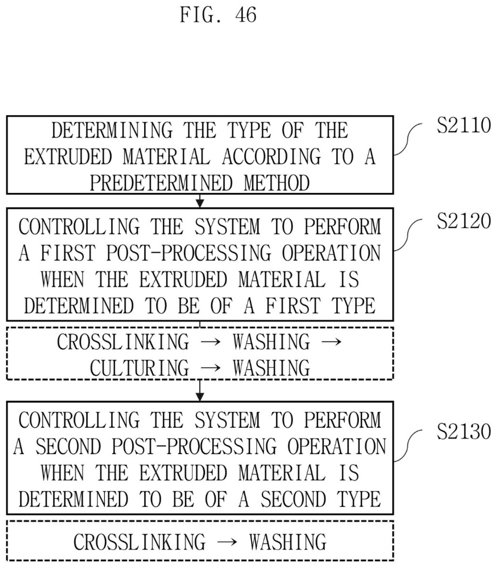

FIG. 45 and FIG. 46 are diagrams illustrating a method by which the processor 2000 controls the post-processing of the extruded material according to an embodiment.

Referring to FIG. 45, the processor 2000 according to an embodiment may control the automated artificial meat production system and may selectively operate at least one of the plurality of processes included in the system based on the type of extruded material.

The processor 2000 may identify the type of extruded material obtained from the plurality of extrusion modules 1300 and control the system so that different post-processing operations are performed based on the type of extruded material. For example, the post-processing operations may include a crosslinking operation performed by the crosslinking module 1410, a washing operation performed by the washing module 1420, and a culturing operation performed by the culturing module 1430. The processor 2000 may control the system so that the post-processing operations corresponding to the extruded material are performed.

Referring to FIG. 46, the processor 2000 may perform an operation of determining the type of the extruded material according to a predetermined method(S2110), an operation of controlling the system to perform a first post-processing operation when the extruded material is determined to be of a first type(S2120), and an operation of controlling the system to perform a second post-processing operation when the extruded material is determined to be of a second type(S2130).

For example, when the processor 2000 determines that the material obtained through the extrusion module 1300 includes cells, it may classify the material as a first type. The processor 2000 may control the system so that the first type of material is post-processed according to a first post-processing process (e.g., a process in which a crosslinking operation, a washing operation, a culturing operation, and a subsequent washing operation are sequentially performed).

In another example, when the processor 2000 determines that the material obtained through the extrusion module 1300 does not include cells, it may classify the material as a second type. The processor 2000 may control the system so that the second type of material is post-processed according to a second post-processing process (e.g., a process in which a crosslinking operation and a washing operation are sequentially performed).

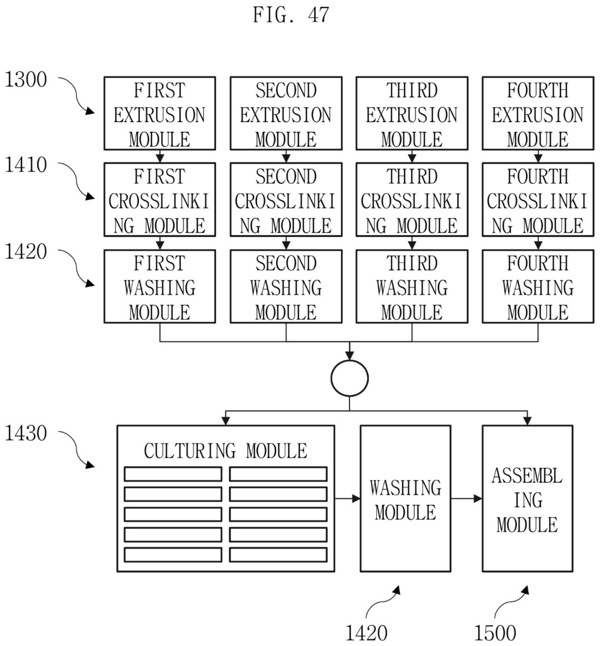

FIG. 47 is a diagram illustrating a method by which the processor controls and supervises a plurality of modules that constitute the automation system according to an embodiment.

Referring to FIG. 47, the automation system according to an embodiment may include at least one extrusion module 1300, at least one crosslinking module 1410, at least one washing module 1420, at least one culturing module 1430, and at least one assembling module 1500.

The processor 2000 may control the plurality of modules constituting the automation system based on control parameters. The processor 2000 may set the control parameters in consideration of factors such as the type of extruded material (e.g., fat-based material, protein-based material, presence or absence of cells) and/or the extrusion timing and may control the plurality of modules constituting the automation system based on these parameters.

The automation system may include a plurality of extrusion modules 1300, and the processor 2000 may control the system such that a first post-processing procedure, which includes passing the extruded materials from the plurality of extrusion modules 1300 through the crosslinking module 1410 and the washing module 1420, is performed.

The processor 2000 may control the system so that a second post-processing procedure is performed on the extruded material that has completed the first post-processing procedure, based on a predetermined criterion, or may control the system so that the material is processed through the assembling module 1500.

The culturing module 1430 may include a plurality of containers. A culture medium mixture may be stored in the plurality of containers, and extruded material obtained from the plurality of extrusion modules may be introduced into any one of the containers for culturing.

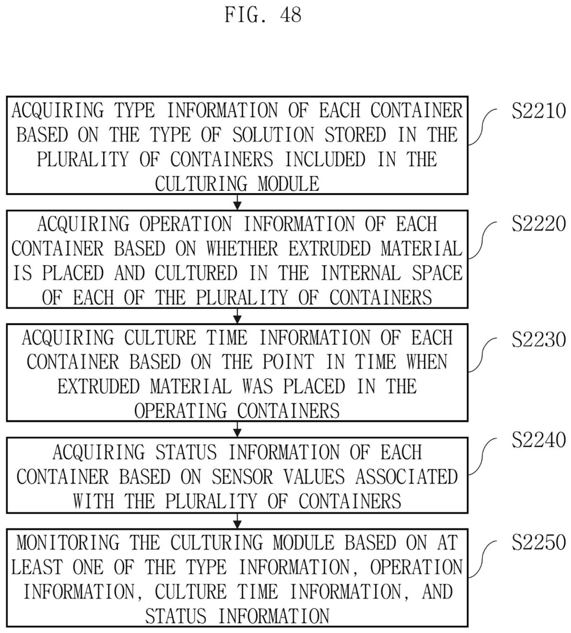

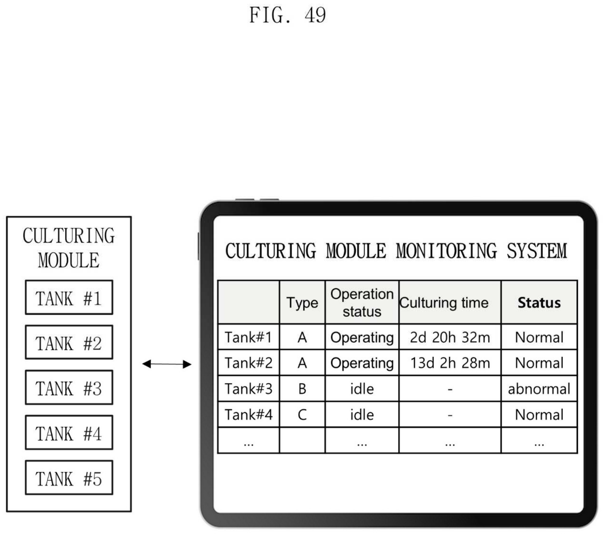

FIG. 48 and FIG. 49 illustrate diagrams for explaining a method by which the processor manages the status of the culturing module according to an embodiment.

Referring to FIGs. 48 and 49, the processor 2000 may perform:
Step 2210: acquiring type information of each container based on the type of solution stored in the plurality of containers included in the culturing module;
Step 2220: acquiring operation information of each container based on whether extruded material is placed and cultured in the internal space of each of the plurality of containers;
Step 2230: acquiring culture time information of each container based on the point in time when extruded material was placed in the operating containers;
Step 2240: acquiring status information of each container based on sensor values associated with the plurality of containers; and
Step 2250: monitoring the culturing module based on at least one of the type information, operation information, culture time information, and status information.

The processor 2000 may acquire type information for each of the plurality of containers based on the composition of the solution stored therein (e.g., amino acid concentration, glucose concentration, temperature, types of additives, etc.).

For example, when a specific type of cell culture is required, the processor 2000 may classify the containers based on the concentration of a specific component in the culture medium mixture. Based on this classification, the processor 2000 may automatically adjust the culturing protocol accordingly.

The processor 2000 may determine whether extruded material is placed in the internal space of each of the plurality of containers and whether culturing is currently in progress, and based on this, the processor 2000 may acquire operation information for each container.

For example, the processor 2000 may use an optical sensor, infrared sensor, or weight-detecting sensor to automatically set containers not currently engaged in culturing to an "inactive state."

The processor 2000 may acquire culturing time information for each of the active containers based on the time at which the extruded material was placed in the container. The processor 2000 may record the culturing start time for each container and based on this, the processor 2000 may acquire the culturing time information. By comparing the culturing start time with the current time, the processor 2000 may acquire the culturing time information in real time. Upon reaching the target culturing duration, the processor 2000 may provide an alert to the user or control the system to proceed with a subsequent process.

The processor 2000 may acquire status information of the containers based on sensor values associated with each of the multiple containers. For example, the processor 2000 may determine whether the culturing environment is being properly maintained by using sensors such as a pH sensor, temperature sensor, dissolved oxygen sensor, conductivity sensor, level sensor, or weight sensor.

Based on at least one of the type information, operation status information, culturing time information, and status information obtained as described above, the processor 2000 may monitor the culturing module 1430.

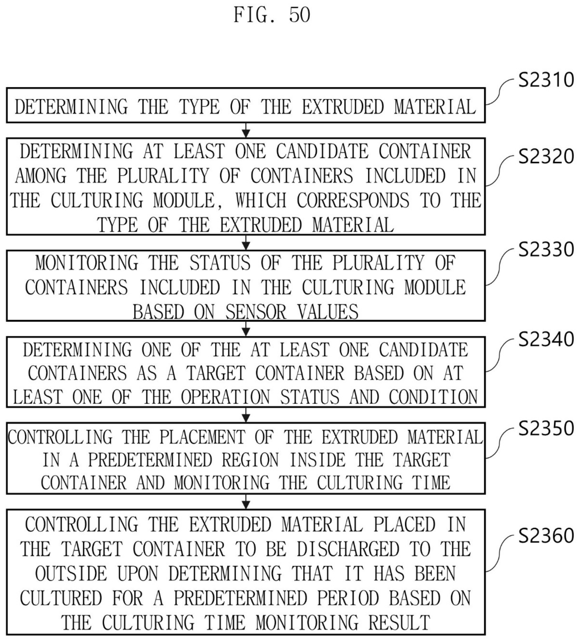

FIG. 50 illustrates a method by which the processor controls a plurality of containers included in the culturing module according to an embodiment.

Referring to FIG. 50, the processor 2000 according to an embodiment may perform an operation of determining the type of the extruded material(S2310). The processor 2000 may determine the type of material based on the physical and/or chemical properties of the extruded material. For example, the processor 2000 may determine the material type based on criteria such as whether it is a protein-based material, fat-based material, hybrid material, the type of cells included, nutritional content, or viscosity.

The processor 2000 may perform an operation of determining at least one candidate container among the plurality of containers included in the culturing module 1430 that corresponds to the type of the extruded material(S2320).

For example, the processor 2000 may determine the candidate container based on criteria such as the composition of the culture medium inside the container, the temperature conditions of the container, the availability status of the container, and the oxygen concentration and pH level within the container.

The processor 2000 may perform an operation of monitoring the status of the plurality of containers included in the culturing module 1430 based on sensor values (e.g., pH, temperature, dissolved oxygen concentration, etc.)(S2330).

The processor 2000 may perform an operation of determining one of the at least one candidate containers as a target container based on at least one of the operation status and condition of the candidate containers(S2340). The processor 2000 may exclude currently operating containers from the candidates and preferentially select containers in an idle state. Based on the sensor values, the processor 2000 may determine the container offering the optimal culturing environment as the target container.

The processor 2000 may perform an operation of controlling the placement of the extruded material in a predetermined region inside the target container and monitoring the culturing time(S2350). Based on the result of the culturing time monitoring, if it is determined that the extruded material has been cultured for a predetermined period, the processor 2000 may perform an operation of controlling the extruded material placed in the target container to be discharged to the outside(S2360).

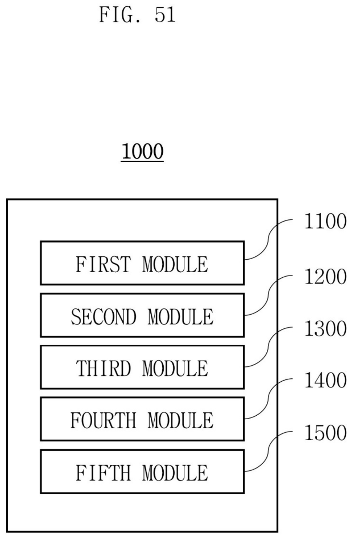

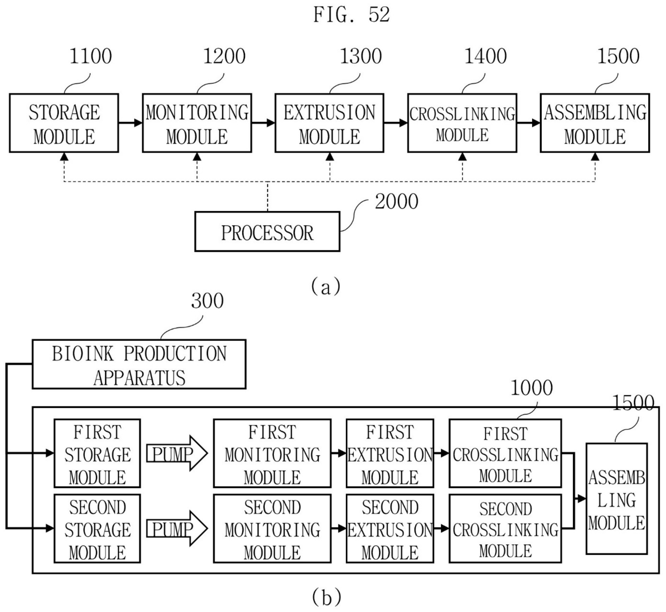

FIG. 51 and FIG. 52 are diagrams illustrating an assembling module according to an embodiment.

Referring to FIG. 51 and FIG. 52, a producing apparatus 100 of the artificial meat manufacturing system according to an embodiment may include a manufacturing module 1000. The manufacturing module 1000 may include a first module 1100, a second module 1200, a third module 1300, a fourth module 1400, and a fifth module 1500. Descriptions of the first to fourth modules (1100 to 1400) are omitted as they have been previously described with reference to FIG. 3.

The fifth module may be an assembling module 1500, and the assembling module 1500 may perform a function of assembling the extruded material (e.g., an extruded fiber bundle) to form the shape of the artificial meat.

The assembling module 1500 may perform operations to precisely position and bind the extruded material, thereby ultimately processing artificial meat that has a tissue structure and appearance similar to actual meat. The assembling module 1500 may include a process of arranging the extruded fiber bundles in a specific pattern and joining each fiber bundle. The joining methods may include physical bonding, chemical bonding, mechanical compression, heat treatment, or the use of specific bio-adhesives.

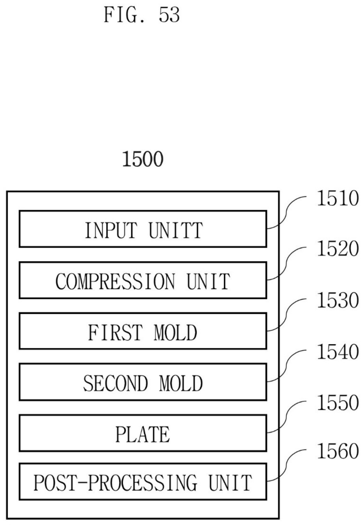

FIG. 53 is a diagram illustrating the configuration of an assembling module according to an embodiment. Referring to FIG. 53, the assembling module 1500 according to the embodiment may include an input unit 1510, a compression unit 1520, a first mold 1530, a second mold 1540, a plate 1550, and a post-processing unit 1560.

The input unit 1510 may perform the function of injecting the extruded material into the mold. For example, the input unit 1510 may apply pressure to the extruded material contained in a syringe so that the extruded material is filled into the mold.

The compression unit 1520 may perform the function of applying physical pressure to the extruded material introduced into the mold so that the extruded material is sufficiently filled in the mold.

The first mold 1530 and the second mold 1540 may be disposed on the plate 1550. The first mold 1530 and the second mold 1540 may provide a space (e.g., a groove) in which the extruded material can be filled and may serve to guide the extruded material so that it can be shaped into a predetermined form.

The first mold 1530 and the second mold 1540 may include a plurality of grooves, and the plurality of grooves may include at least one groove having a width different from the others. The first mold 1530 and the second mold 1540 may include a plurality of grooves with adjustable widths. The plurality of grooves may be formed such that the respective widths are adjustable according to predetermined criteria.

The first mold 1530 and the second mold 1540 may be provided in the form of replaceable modules to allow changes in shape. When the replaceable module form is applied, the first mold 1530 and the second mold 1540 can be used to produce artificial meat in various sizes and shapes.

The first mold 1530 and the second mold 1540 may be intended for processing different types of materials. For example, the first mold 1530 may be used for assembling a first material, and the second mold 1540 may be used for assembling a second material.

The post-processing unit 1560 may perform a post-processing function on at least a portion of the extruded material filled in the first mold 1530 and the second mold 1540. For example, the post-processing unit 1560 may perform a cutting function (or trimming function) on at least a portion of the extruded material filled in the first mold 1530 and the second mold 1540. More specifically, when the input unit 1510 feeds the material in a first direction, the post-processing unit 1560 may perform a cutting operation in a second direction perpendicular to the first direction.

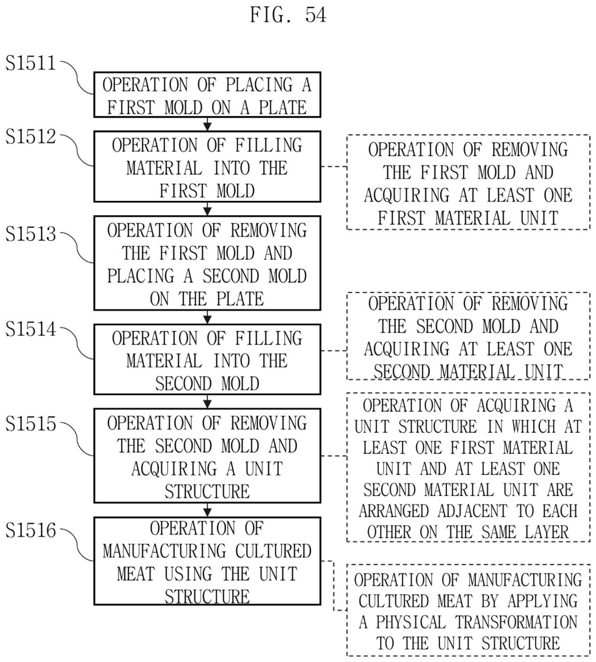

FIG. 54 through FIG. 56 are diagrams illustrating a first method by which the assembling module manufactures cultured meat according to an embodiment.

Referring to FIG. 54, the assembling module 1500 according to an embodiment may perform: an operation of placing a first mold on a plate (S1511), an operation of filling material into the first mold (S1512), an operation of removing the first mold and placing a second mold on the plate (S1513), an operation of filling material into the second mold (S1514), an operation of removing the second mold and acquiring a unit structure (S1515) and an operation of manufacturing cultured meat using the unit structure (S1516).

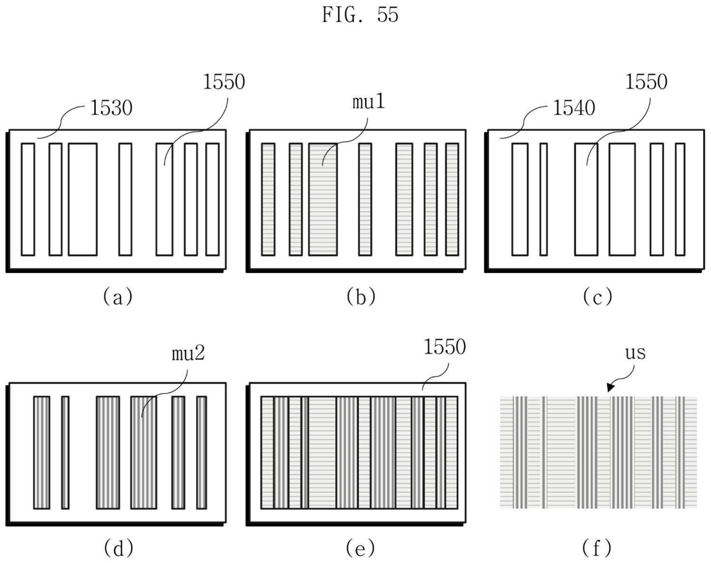

The assembling module 1500 may perform an operation of placing the first mold 1530 on the plate 1550, as shown in FIG. 55(a).

The assembling module 1500 may perform an operation of filling the extruded material into the first mold 1530, as shown in FIG. 55(b). The assembling module 1500 may fill the grooves of the first mold 1530 with the extruded material using the input unit 1510. For example, the assembling module 1500 may perform the operation of filling the first mold 1530 with the extruded material using the input unit 1510 that operates based on an air cylinder mechanism.

The material filled in the grooves of the first mold 1530 may be a first material unit (mu1), and the first material unit mu1 may correspond in number and shape to the grooves included in the first mold 1530. For example, the first material unit mu1 may be a material unit corresponding to fat.

The assembling module 1500 may perform an operation of removing the first mold 1530 and placing the second mold 1540 on the plate 1550, as shown in FIG. 55(c). The assembling module 1500 may leave the first material unit (mu1) obtained through the first mold 1530 on the plate 1550 while removing only the first mold 1530. The assembling module 1500 may place the second mold 1540 on the plate 1550 and over the first material unit(mu1).

The assembling module 1500 may perform an operation of filling the second mold 1540 with an extruded material, as shown in FIG. 55(d). The extruded material filled through the second mold 1540 may be of a different type from the extruded material filled through the first mold 1530. The material may be filled into the grooves of the second mold 1540. For example, the assembling module 1500 may fill the second mold 1540 with the extruded material using the injection unit 1510, which operates based on an air cylinder mechanism.

The material filled in the grooves of the second mold 1540 may be a second material unit (mu2), and the second material unit (mu2) may correspond in number and shape to the grooves included in the second mold 1540. For example, the second material unit (mu2) may be a material unit corresponding to protein.

The assembling module 1500 may perform an operation of removing the second mold 1540 and acquiring a unit structure (us), as illustrated in FIG. 55(e) and FIG. 55(f). The unit structure (us) may be a combination of at least one first material unit (mu1) and at least one second material unit (mu2).

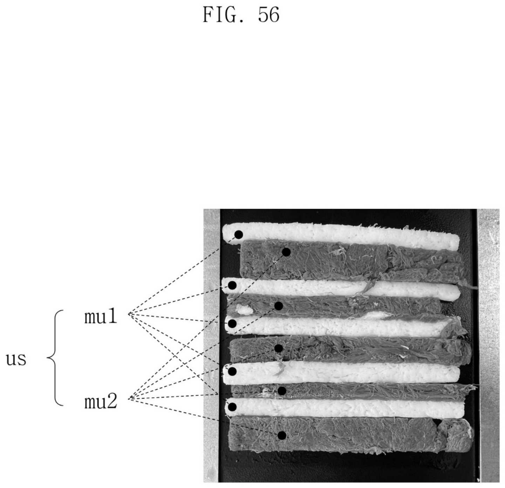

Referring to FIG. 56, the unit structure (us) may be composed of at least one first material unit (mu1) and at least one second material unit (mu2) that are arranged adjacent to each other on the same layer. The unit structure (us) may be configured such that at least one first material unit (mu1) and the at least one second material unit (mu2) are arranged in an intersecting pattern.

The assembling module 1500 may perform an operation of manufacturing artificial meat using the unit structure (us). The assembling module 1500 may perform an operation of manufacturing the artificial meat by applying physical modifications to the unit structure (us). The operation of manufacturing the artificial meat may include processing the unit structure (us) into a shape like actual meat.

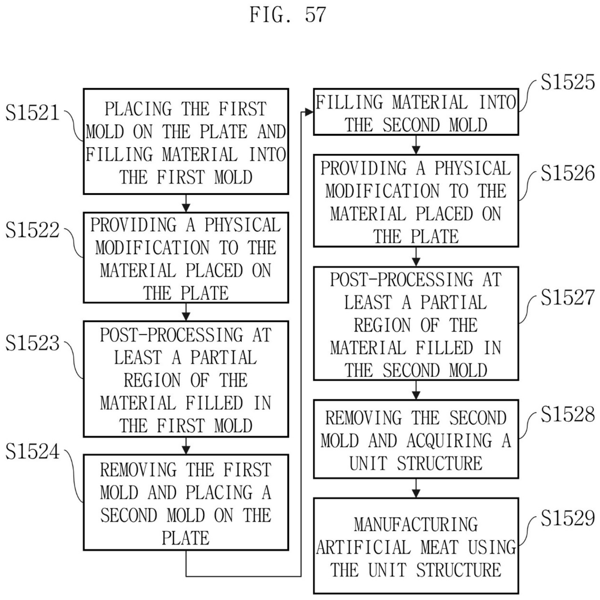

FIG. 57 illustrates a second method of manufacturing artificial meat using the assembling module according to an embodiment.

Referring to FIG. 57, the assembling module 1500 according to one embodiment may perform the following operations: an operation of placing a first mold on a plate and filling material into the first mold (S1521); an operation of applying a physical modification to the material placed on the plate (S1522); an operation of post-processing at least a partial region of the material filled in the first mold (S1523); an operation of removing the first mold and placing a second mold on the plate (S1524); an operation of filling material into the second mold (S1525); an operation of applying a physical modification to the material placed on the plate (S1526); an operation of post-processing at least a partial region of the material filled in the second mold (S1527); an operation of removing the second mold and acquiring a unit structure (S1528); and an operation of manufacturing artificial meat using the unit structure (S1529).

Some of the above operations (e.g., S1521, S1524, S1525, S1528, and S1529) correspond to the operations previously described with reference to FIG. 54, and thus redundant explanations will be omitted.

The assembling module 1500 may perform an operation of applying a physical change to the material after the material is filled into the first molding mold 1530. The assembling module 1500 may apply a physical change (e.g., compression) to the material filled in the first molding mold 1530 via the compression unit 1520. By applying the physical change to the material filled in the first molding mold 1530, the material can be more densely packed in the grooves of the first molding mold 1530. Likewise, the assembling module 1500 may perform an operation of applying a physical change to the material after the material is filled into the second molding mold 1540. Since this corresponds to the above-described content, redundant explanations will be omitted.

The assembling module 1500 may perform a post-processing operation (e.g., a cutting operation) on at least a partial region of the material filled in the first molding mold 1530. By performing a post-processing operation (e.g., cutting) on at least a portion of the material, the assembling module 1500 can obtain at least one first material unit mu1 corresponding to the grooves of the first molding mold 1530. The assembling module 1500 may also perform a post-processing operation (e.g., cutting) on at least a partial region of the material filled in the second molding mold 1540. Since this corresponds to the above-described content, redundant explanations will be omitted.

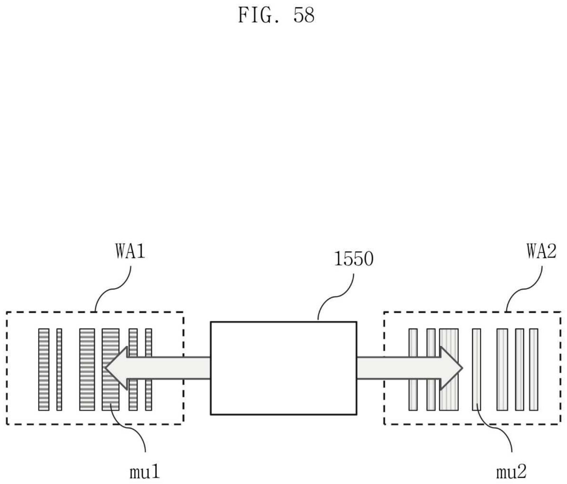

FIG. 58 is a diagram illustrating a method in which the assembling module operates in a plurality of working areas according to an embodiment.

Referring to FIG. 58, the assembling module 1500 according to an embodiment may obtain unit structures us in a plurality of working areas. The assembling module 1500 may be configured to obtain at least one first material unit mu1 in a first working area WA1, and to obtain at least one second material unit mu2 in a second working area WA2.

The assembling module 1500 may control the plate 1550 to sequentially move between the first working area WA1 and the second working area WA2. The assembling module 1500 may obtain at least one first material unit mu1 and at least one second material unit mu2 at different locations.

The assembling module 1500 may include a pair of injection units 1510. The pair of injection units 1510 may include a first injection unit 1510a and a second injection unit 1510b, wherein the first injection unit 1510a may be disposed in the first working area WA1, and the second injection unit 1510b may be disposed in the second working area WA2.

The first injection unit 1510a may be a device for injecting the first material, and the second injection unit 1510b may be a device for injecting the second material. The assembling module 1500 may obtain at least one first material unit mu1 through the first injection unit 1510a in the first working area WA1 and may obtain at least one second material unit mu2 through the second injection unit 1510b in the second working area WA2.

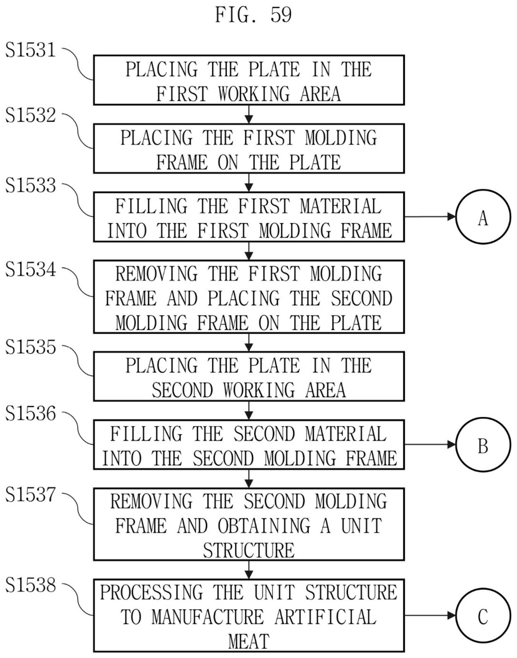

FIGS. 59 to 61 are diagrams illustrating a third method for manufacturing cultured meat using an assembling module according to an embodiment.

Referring to FIG. 59, the assembling module 1500 according to an embodiment may perform operations including: an operation of positioning a plate in a first working area(S1531 ); an operation of placing a first molding frame on the plate(S1532); an operation S1533 of filling a first material into the first molding frame(S1533); an operation of removing the first molding frame and placing a second molding frame on the plate(S1534); an operation of positioning the plate in a second working area(S1535); an operation of filling a second material into the second molding frame(S1536); an operation of removing the second molding frame and obtaining a unit structure(S1537); and an operation of manufacturing cultured meat using the unit structure(S1538).

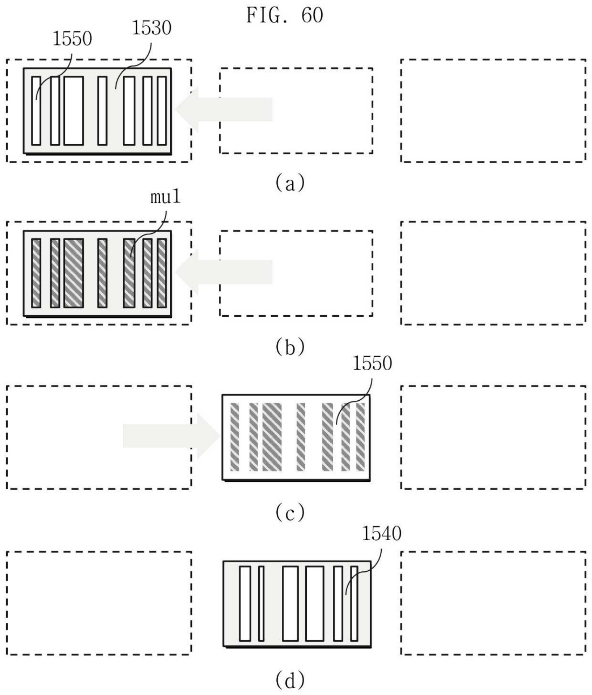

As shown in FIG. 60(a), the assembling module 1500 may perform an operation of positioning the plate 1550 in the first working area WA1 and placing the first molding frame 1530 on the plate 1550.

As shown in FIG. 60(b) and FIG. 60(c), the assembling module 1500 may perform an operation of filling a first material into the first molding frame 1530 placed in the first working area WA1. The assembling module 1500 may fill the first material into the first molding frame 1530 through the first input unit 1510a. Through this operation, the assembling module 1500 may obtain at least one first material unit mu1.

Although not illustrated in the drawings, the assembling module 1500 may obtain at least one first material unit (mu1) by performing an operation of applying a physical change (e.g., compression) to the first material after it is filled into the first molding frame 1530 and/or by performing a post-processing operation (e.g., cutting) on at least a portion of the first material.

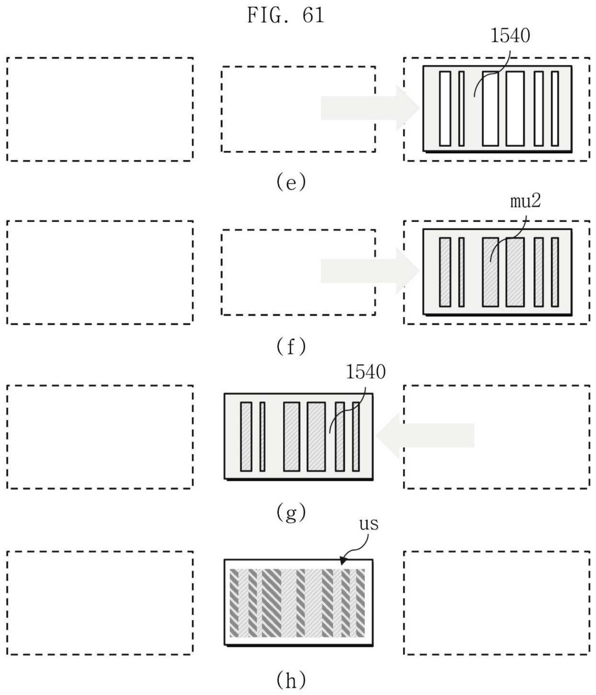

As illustrated in FIG. 60(d), the assembling module 1500 may perform an operation of removing the first molding frame 1530 and placing the second molding frame 1540 on the plate 1550. As shown in FIG. 61(e), the assembling module 1500 may perform an operation of positioning the plate 1550 in the second work area WA2. Although not illustrated in the drawings, the operation of placing the second molding frame 1540 on the plate 1550 may be performed after positioning the plate 1550 in the second work area WA2.

As illustrated in FIG. 61(f) and FIG. 61(g), the assembling module 1500 may perform an operation of filling the second molding frame 1540 with a second material. The assembling module 1500 may fill the second molding frame 1540 with the second material through the second injector 1510b. Through this filling operation, the assembling module 1500 may obtain at least one second material unit mu2.

As not illustrated in the drawings, the assembling module 1500 may perform an operation of applying a physical change (e.g., compression) to the second material after it is filled into the second molding frame 1540, and/or an operation of post-processing at least a portion of the second material (e.g., cutting), thereby obtaining at least one second material unit (mu2).

As shown in FIG. 61(h), the assembling module 1500 may perform an operation of removing the second molding frame 1540 and obtaining a unit structure (us). The assembling module 1500 may also perform an operation of processing the unit structure (us) according to a predetermined method to manufacture artificial meat.

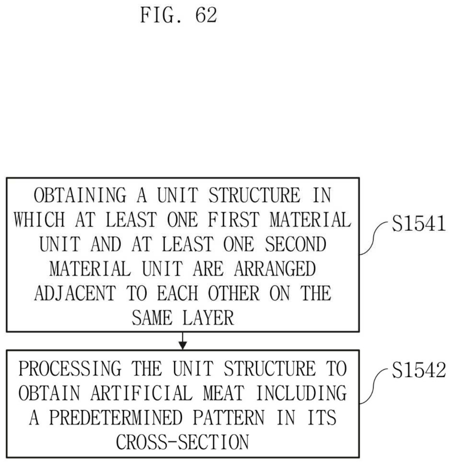

FIG. 62 through FIG. 64 are diagrams illustrating a method by which the assembling module manufactures artificial meat having a predetermined pattern according to an embodiment.

Referring to FIG. 62, the assembling module 1500 according to an embodiment may perform a step S1541 of obtaining a unit structure in which at least one first material unit and at least one second material unit are arranged adjacent to each other on the same layer, and a step S1542 of processing the unit structure to obtain artificial meat that includes a predetermined pattern in its cross-section.

The assembling module 1500 may process the unit structure in which at least one first material unit and at least one second material unit are arranged adjacent to each other on the same layer to obtain artificial meat. The assembling module 1500 may process the unit structure to manufacture artificial meat in which a user-desired pattern appears in the cross-section.

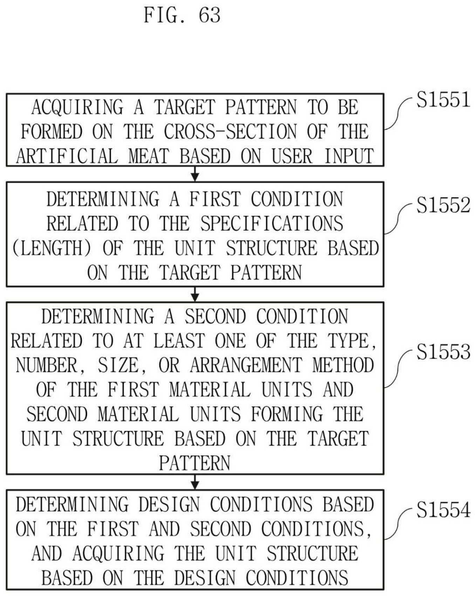

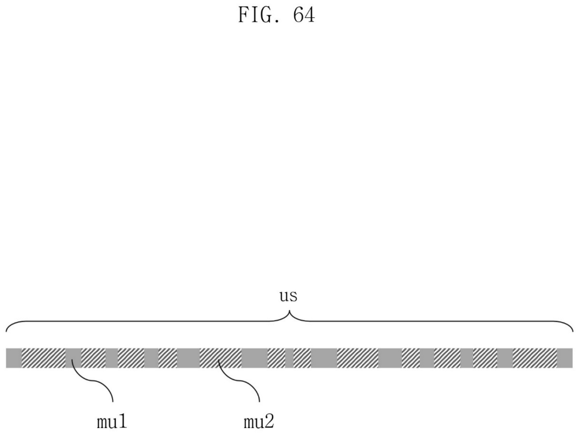

Referring to FIG. 63 and FIG. 64, the assembling module 1500 according to an embodiment may perform the steps of: acquiring a target pattern to be formed on the cross-section of the artificial meat based on a user input(S1551); determining a first condition related to the specifications (length) of the unit structure based on the target pattern(S1552); determining a second condition related to at least one of the type, number, size, or arrangement method of the first material units and the second material units forming the unit structure based on the target pattern(S1553); and determining design conditions based on the first and second conditions, and acquiring the unit structure based on the design conditions(S1554).

The assembling module 1500 may acquire a target pattern to be formed on the cross-section of the artificial meat based on a user input or a predefined dataset. The target pattern may include the muscle fiber alignment of actual meat, marbling (i.e., the mixture pattern of muscle and fat), tissue density, and directional characteristics, and may be a custom-designed pattern for specific aesthetic or functional purposes.

The assembling module 1500 may determine a first condition related to at least one of the dimensions, length, and width of the unit structure us based on the target pattern. The assembling module 1500 may also determine a second condition related to at least one of the type, quantity, size, and arrangement method of at least one first material unit mu1 and at least one second material unit mu2 that constitute the unit structure us, based on the target pattern. The first and second conditions may be determined in consideration of the size of the artificial meat, the shape of its cross-section, and the minimum dimensions required to implement the target pattern.

The assembling module 1500 may determine design conditions based on the first condition and/or the second condition and may obtain the unit structure us based on the design conditions.

The assembling module 1500 may apply a predetermined process-such as a rolling process, a cylindrical winding process, or a compression process-to the obtained unit structure us, thereby manufacturing artificial meat in which the target pattern is formed on the cross-section.

The unit structure us may include a plurality of first material units mu1 and a plurality of second material units mu2, and the plurality of first material units mu1 and the plurality of second material units mu2 may be alternately arranged (e.g., in a cross pattern) on the same layer.

The assembly module 1500 may control the widths of grooves (slots) forming the first molding frame 1530 and/or the second molding frame 1540 to be adjusted so as to meet the first condition and/or the second condition. Artificial meat with a target pattern formed on a cross-section may be obtained by using the first molding frame 1530 and the second molding frame 1540 adjusted according to the first condition and the second condition.

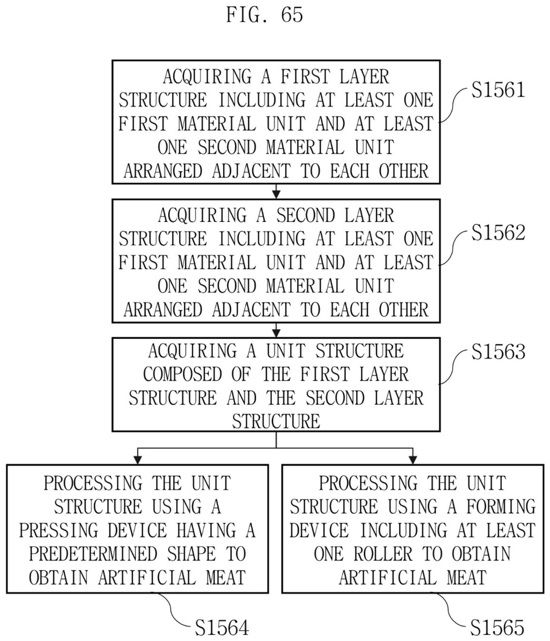

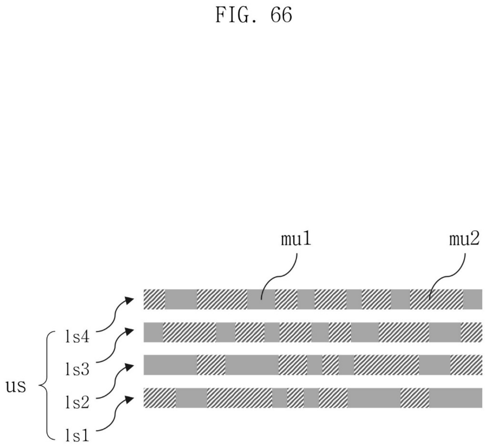

FIG. 65 and FIG. 66 illustrate a method in which the assembly module 1500 processes a unit structure including a plurality of layers to manufacture artificial meat, according to an embodiment.

Referring to FIG. 65, the assembly module 1500 according to an embodiment may perform the following steps:
Step 1561: Acquiring a first layer structure including at least one first material unit and at least one second material unit arranged adjacent to each other;
Step 1562: Acquiring a second layer structure including at least one first material unit and at least one second material unit arranged adjacent to each other;
Step 1563: Acquiring a unit structure composed of the first layer structure and the second layer structure;
Step 1564: Processing the unit structure using a pressing device having a predetermined shape to obtain artificial meat;
Step 1565: Processing the unit structure using a forming device including at least one roller to obtain artificial meat.

Here, the artificial meat acquisition steps (S1564 and S1565) may be performed selectively.

Referring to FIG. 66, the assembling module 1500 may acquire a unit structure (us), which may include a plurality of layer structures (e.g., the first layer structure(ls 1) to the fourth layer structure(ls4)).

The first layer structure (ls1) may include at least one first material unit (mu1) and at least one second material unit (mu2), and the second layer structure (ls2) may also include at least one first material unit (mu1) and at least one second material unit (mu2). The first layer structure (ls1) and the second layer structure (ls2) may be arranged vertically to form the unit structure (us). The first layer structure (ls1) and the second layer structure (ls2) may be assembled by being stacked in the vertical direction.

For example, the plurality of layer structures may have different properties. For instance, the first layer structure (ls1) may be a high-protein fiber layer, the second layer structure (ls2) may be a fat layer, and the third layer structure (ls3) may be a low-density muscle layer. Through such customized layering, it is possible to artificially implement a structure similar to specific cuts of meat (e.g., sirloin, tenderloin, etc.).

The assembling module 1500 may manufacture cultured meat by processing the unit structure(us) composed of a plurality of layers obtained through the above method. The assembling module 1500 may apply pressure to the unit structure(us) using a pressing device having a predetermined shape to obtain the cultured meat.

The assembling module 1500 may process the unit structure (us) using a forming device including at least one roller to obtain the cultured meat. The forming device may include a pair of rollers, such as an upper roller and a lower roller. Alternatively, the forming device may include only the upper roller.

The cultured meat manufactured by processing the unit structure (us) including a plurality of layers may reproduce a meat-like texture through layer-by-layer alignment of muscle fibers, enhance strength and elasticity due to increased interlayer bonding force, and enable more precise marbling formation through optimal arrangement of fat and protein layers.

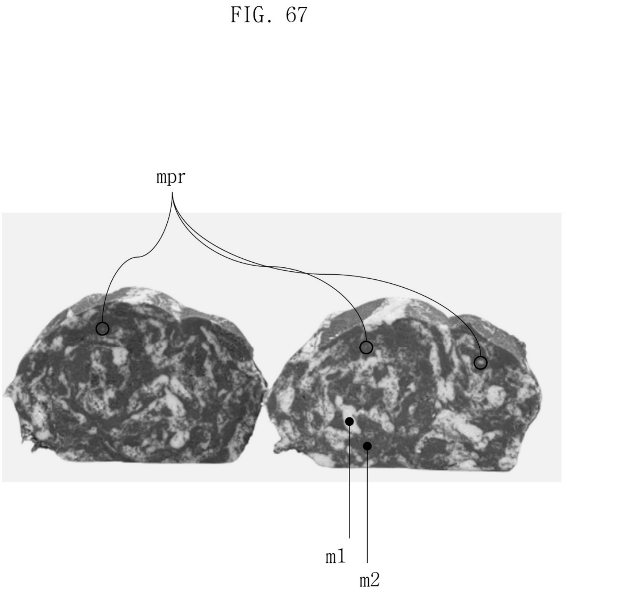

FIG. 67 is a diagram illustrating a cross-section of cultured meat manufactured by the producing apparatus according to an embodiment. Referring to FIG. 67, the producing apparatus 100 according to the embodiment may manufacture cultured meat in which a pattern is formed on the cross-section.

The pattern may include, as described above, muscle fiber arrangements, marbling (a combination of muscle and fat), tissue density, and tissue orientation, and may be a custom-designed pattern for specific aesthetic or functional purposes.

The producing apparatus 100 may process the first material and the second material to obtain artificial meat, and a pattern may be formed in the artificial meat by the first material and the second material.

The producing apparatus 100 may extrude the first material in a longitudinal direction to obtain a first microstructure m1 and extrude the second material in a longitudinal direction to obtain a second microstructure m2. The longitudinal direction may correspond to the direction in which pressure is applied. More specifically, the producing apparatus 100 may process the first material through an extrusion method using at least one nozzle to obtain the first microstructure m1 and may process the second material to obtain the second microstructure m2.

The pattern may be formed by at least one first microstructure m1 obtained by processing the first material and at least one second microstructure m2 obtained by processing the second material.

The pattern may be determined by at least one of the number, the arrangement (e.g., parallel arrangement, cross arrangement, random arrangement, etc.), and the coupling method (e.g., method of assembling microstructures, compression strength, etc.) of the at least one first microstructure m1.

The pattern may be formed by at least one first microstructure m1, and an outer boundary of a region defined by the pattern may be formed to contact at least one second microstructure m2. For example, the region defined by the pattern may be surrounded by a plurality of second microstructures m2.

The producing apparatus 100 may obtain at least one first microstructure m1 and at least one second microstructure m2 by extruding a first material and a second material, respectively, in a longitudinal direction within a first working space. The producing apparatus 100 may assemble the at least one first microstructure m1 and the at least one second microstructure m2 through physical and/or chemical bonding methods within a second working space. Here, the first working space and the second working space may be separate spaces.

The producing apparatus 100 may obtain at least one first microstructure m1 and at least one second microstructure m2 by extruding a first material and a second material, respectively, in a longitudinal direction at a first point in time. The producing apparatus 100 may assemble the at least one first microstructure m1 and the at least one second microstructure m2 through physical and/or chemical bonding methods at a second point in time. Here, the first point in time and the second point in time may be different.

For example, according to one embodiment, the producing apparatus 100 may obtain microstructures through an extrusion process, perform the aforementioned post-processing step, and then acquire artificial meat having a pattern formed on its cross-section through an assembling process of the microstructures.

The cross-section of the artificial meat produced by the producing apparatus 100 may include a plurality of patterns, and the plurality of patterns may exhibit a certain periodicity and may be formed in different shapes.

Among the plurality of patterns included in the cross-section of the artificial meat, at least one may have a minimum width of 1 mm to 2 mm or less. Regardless of the geometric shapes of the plurality of patterns, at least one of the patterns may have a minimum width (i.e., minimum cross-sectional dimension) formed to be 1 mm to 2 mm or less.

The plurality of patterns included in the cross-section of the artificial meat may have various geometric shapes. However, the minimum width of individual structural elements constituting at least one of the plurality of patterns may be formed to be 1 mm to 2 mm or less.

The cross-section of the artificial meat may include a plurality of patterns having a plurality of measurable widths (e.g., minimum width, minimum cross-sectional dimension, minimum feature size, any one of the geometric elements within the pattern, or the width of the narrowest spatial portion). At least one of the plurality of measurable widths may be formed to be 1 mm to 2 mm or less.

For example, at least one of the plurality of patterns may have a polygonal shape, in which a plurality of measurable widths within the polygonal shape may be formed to be 1 mm to 2 mm or less. In another example, at least one of the plurality of patterns may have a circular shape, in which the minimum diameter of the circular shape may be formed to be 1 mm to 2 mm or less.

The cross-section of the artificial meat manufactured by the producing apparatus 100 may include a plurality of pattern regions defined by a plurality of patterns. At least one of the plurality of pattern regions may be a minimum pattern region.

The minimum pattern region may be a pattern structure formed to have specific physical or visual characteristics within the cross-section of the artificial meat, and may have an area below a certain size. The minimum pattern region may be defined by a pattern formed below a predetermined size. The minimum width of a pattern located within the minimum pattern region may be 1 mm to 2 mm or less.

The minimum pattern region may include a feature region formed by at least one first microstructure, and a background region in which the outer boundary line of the feature region is in contact with at least one second microstructure.

The minimum pattern region may be defined by a predetermined guide tool. The guide tool may serve to distinguish patterns of a certain size or smaller on the cross-section of the cultured meat and define them as minimum pattern regions. In other words, the guide tool functions as a filter based on a specified pattern size, whereby patterns equal to or smaller than the threshold size are determined to be minimum pattern regions.

Among the multiple patterns included in the cross-section of the cultured meat, those that are distinguished as being below a certain size by the guide tool may be defined as minimum pattern regions. The guide tool may define a projection area of a predetermined size, and among the multiple patterns included in the cross-section of the cultured meat, the patterns detected within the defined projection area may be defined as minimum pattern regions. Patterns located within the projection area set by the guide tool may be defined as minimum pattern regions.

For example, the guide tool may be circular, and the diameter of the circle may be 1 mm or less, or 2 mm or less. In this case, the cross-section of the cultured meat manufactured by the producing apparatus 100 may include at least one minimum pattern region that is located within the projection area defined by the guide tool (i.e., an area having a diameter of 1 mm or less to 2 mm or less).

The features, structures, and effects described in the above embodiments are included in at least one embodiment of the present disclosure but are not necessarily limited to a single embodiment. The features, structures, and effects exemplified in each embodiment may be combined or modified with other embodiments by those skilled in the art. Therefore, such combinations and modifications are considered within the scope of the present disclosure.

While specific embodiments have been described, it should be understood that these embodiments are exemplary and not limiting. People skilled in the art will recognize that various modifications and applications can be made without departing from the spirit and scope of the invention. Such modifications and variations are considered to be within the scope of the present disclosure as defined by the appended claims.

## Claims

1. An apparatus for producing artificial meat using bio-ink, the apparatus comprising:
a plurality of modules; and
at least one processor configured to control the plurality of modules,
wherein the plurality of modules includes a monitoring module and an extrusion module, and
wherein the at least one processor is configured to:
control the monitoring module to maintain a predetermined condition of the bio-ink, and
control the extrusion module to extrude and discharge at least a portion of the bio-ink.

2. The apparatus of claim 1,
wherein the monitoring module includes an inner space configured to accommodate the bio-ink and at least one sensor configured to monitor a state of the bio-ink accommodated in the inner space, and
wherein the at least one sensor includes a first sensor configured to measure the temperature of the bio-ink or a second sensor configured to measure a pH of the bio-ink.

3. The apparatus of claim 2,
wherein the extrusion module further includes a nozzle having a plurality of holes,
wherein the at least one processor is configured to control the extrusion module such that at least a portion of the bio-ink is extracted in a form of a fiber bundle through the nozzle.

4. The apparatus of claim 3,
wherein each of the plurality of holes formed in the nozzle is formed to have a predetermined identical diameter, is spaced apart from each other by a predetermined interval, and is formed to have a Y-shaped cross-section.

5. The apparatus of claim 4,
wherein the apparatus further comprises a crosslinking module,
wherein the crosslinking module includes an internal space capable of containing a crosslinking liquid, and
wherein the at least one processor is configured to control the bio-ink extruded and discharged through the extrusion module to be introduced into the internal space of the crosslinking module.

6. The apparatus of claim 5,
wherein the bio-ink comprises a first bio-ink related to fat and a second bio-ink related to protein,
wherein the monitoring module comprises a first monitoring module for monitoring the first bio-ink and a second monitoring module for monitoring the second bio-ink,
wherein the extrusion module comprises a first extrusion module for extruding the first bio-ink and a second extrusion module for extruding the second bio-ink, and
wherein the crosslinking module comprises a first crosslinking module into which the first bio-ink is introduced after being discharged, and a second crosslinking module into which the second bio-ink is introduced after being discharged.

7. The apparatus of claim 6,
wherein the plurality of modules comprise a first unit and a second unit,
wherein the first unit includes the first monitoring module, the first extrusion module, and the first crosslinking module,
wherein the second unit includes the second monitoring module, the second extrusion module, and the second crosslinking module, and
wherein the at least one processor is configured to control the first unit and the second unit in parallel.

8. The apparatus of claim 5,
wherein the crosslinking module includes a conductivity sensor,
wherein the apparatus is configured to measure a contamination concentration of the crosslinking liquid accommodated in the internal space of the crosslinking module based on the conductivity sensor, and
when the contamination concentration is determined to exceed a predetermined level, the apparatus is configured to control the crosslinking liquid to be circulated and replaced.

9. The apparatus of claim 5,
wherein the apparatus further comprises a storage module,
wherein the storage module includes an internal space for storing the bio-ink, and
wherein the at least one processor is configured to control at least a portion of the bio-ink accommodated in the internal space of the storage module to be transferred to the monitoring module via a pump.

10. A method for producing artificial meat using bio-ink, the method comprising:
storing the bio-ink in an internal space of a storage module;
transferring at least a portion of the bio-ink stored in the internal space of the storage module to a monitoring module via a pump;
controlling the state of the bio-ink stored in the monitoring module to maintain a predetermined condition; and
controlling an extrusion module to extrude and discharge at least a portion of the bio-ink stored in the monitoring module.
